# EUROPEAN PATENT APPLICATION

(11) **EP 4 840 387 A1**
(43) Date of publication of application: **18.11.2026**
(21) Application number: 24917094.5
(22) Date of filing: 23.08.2024
(51) Int. Cl.: A61B 3/10

(54) **OPHTHALMOLOGIC SYSTEM**

(30) Priority: 10.01.2024 JP 2024002163
(71) Applicant: Topcon Corporation, Tokyo 174-8580 (JP)
(72) Inventor: OMIYA, Takeshi, Tokyo 174-8580 (JP)
(74) Representative: Louis Pöhlau Lohrentz
(86) International application number: PCT/JP2024/029958
(87) International publication number: WO 2025/150219

(57) **Abstract**

An ophthalmic system is provided which, when an examiner remotely operates a plurality of ophthalmic apparatuses using one remote operation apparatus, enables the examiner to individually communicate with a plurality of subjects. The ophthalmic system includes three ophthalmic apparatuses and one remote operation apparatus provided at a location away from each of the ophthalmic apparatuses. Each of the ophthalmic apparatuses and the remote operation apparatus are configured to communicate with one another, and an examiner remotely operates each of the ophthalmic apparatuses using the remote operation apparatus. The ophthalmic apparatuses and the remote operation apparatus each include a microphone and a speaker. The ophthalmic system has an interactive function that enables a subject at each of the ophthalmic apparatuses and an examiner at the remote operation apparatus to communicate with one another. When the interactive function is enabled in two or more ophthalmic apparatuses among the plurality of ophthalmic apparatuses (Step S101), the remote operation apparatus is configured to maintain the enabled state of the interactive function in one of the ophthalmic apparatuses (Step S102 and other steps) and issue an instruction to a remainder of the ophthalmic apparatuses to pause the interactive function thereof (Step S103).

## Description

### Technical Field

The present disclosure relates to an ophthalmic system.

### Background Art

Ophthalmic systems are known which enable an examiner (such as an ophthalmologist, an optometrist, or an orthoptist) to remotely operate an ophthalmic apparatus (hereinafter this may be abbreviated as "remote operation by the examiner") using a host apparatus via a network such as the Internet (for example, refer to Patent Literatures 1 and 2). In addition, an ophthalmic system is known in which, when remote operation by the examiner is enabled, the host apparatus includes a microphone and the ophthalmic apparatus includes a speaker to allow the examiner's voice to be transmitted to a subject through the speaker (for example, refer to Patent Literature 2).

### Citation List

### Patent Literature

Patent Literature 1: JP 2022-073686 A
Patent Literature 2: JP 2023-039506 A

### Summary of Invention

### Technical Problem

However, in a remote operation by the examiner, the subject may want to communicate with the examiner. In addition, since the remote operation by the examiner is enabled, the examiner can remotely operate a plurality of ophthalmic apparatuses using one host apparatus. As a result, a problem arises in that communicating with a plurality of subjects at the same time makes it difficult for one examiner to communicate appropriately.

The present disclosure has been made in view of the problem described above. An object of the present disclosure is to provide an ophthalmic system that enables one examiner to communicate individually with a plurality of subjects when remotely operating a plurality of ophthalmic apparatuses using one host apparatus.

### Solution to Problem

The ophthalmic system according to the present disclosure includes a plurality of ophthalmic apparatuses and a host apparatus provided at a location away from each of the ophthalmic apparatuses. Each of the plurality of ophthalmic apparatuses and the host apparatus are configured to communicate with one another, and an examiner remotely operates each of the plurality of ophthalmic apparatuses using the host apparatus. The plurality of ophthalmic apparatuses and the host apparatus each include a microphone and a speaker. The ophthalmic system includes an interactive function that enables a subject at each of the ophthalmic apparatuses and an examiner at the host apparatus to communicate with one another. When the interactive function is enabled in two or more ophthalmic apparatuses among the plurality of ophthalmic apparatuses, the host apparatus is configured to maintain the enabled state of the interactive function in one of the ophthalmic apparatuses and issue an instruction to a remainder of the ophthalmic apparatuses to pause the interactive function thereof.

### Advantageous Effect of Invention

According to the ophthalmic system of the present disclosure, when an examiner remotely operates a plurality of ophthalmic apparatuses using one host apparatus, the examiner can individually communicate with a plurality of subjects.

### Brief Description of Drawings

[FIG. 1] FIG. 1 is a schematic diagram showing connections between one remote operation apparatus and a plurality of ophthalmic apparatuses in an ophthalmic system as a first embodiment of the present disclosure.
[FIG. 2] FIG. 2 is a schematic diagram showing an example of the ophthalmic system.
[FIG. 3] FIG. 3 is a block diagram showing a configuration of a control system in the ophthalmic system.
[FIG. 4] FIG. 4 is a schematic diagram showing an example of subjects at three ophthalmic apparatuses displayed on an examiner monitor portion.
[FIG. 5] FIG. 5 is a flow chart showing a flow of processing by each ophthalmic apparatus included in the ophthalmic system.
[FIG. 6] FIG. 6 is a sub-flow chart showing examination guidance processing by each ophthalmic apparatus.
[FIG. 7] FIG. 7 is a sub-flow chart showing examination execution processing by each ophthalmic apparatus.
[FIG. 8] FIG. 8 is a flow chart showing execution processing by the remote operation apparatus when the interactive function is enabled.
[FIG. 9] FIG. 9 is a sub-flow chart showing execution processing by an ophthalmic apparatus when the interactive function is enabled.
[FIG. 10] FIG. 10 is a time chart explaining an example of processing of control performed in the flow charts of FIGS. 8 and 9 according to the first embodiment.
[FIG. 11A] FIG. 11A is a schematic diagram showing an example displayed on the examiner monitor portion at time point t1 of the time chart shown in FIG. 10.
[FIG. 11B] FIG. 11B is a schematic diagram showing an example displayed on the examiner monitor portion at time point t2 of the time chart shown in FIG. 10.
[FIG. 11C] FIG. 11C is a schematic diagram showing an example displayed on the examiner monitor portion at time point t6 of the time chart shown in FIG. 10.
[FIG. 11D] FIG. 11D is a schematic diagram showing an example displayed on the examiner monitor portion at time point t7 of the time chart shown in FIG. 10.

### Description of Embodiments

Embodiments for implementing the ophthalmic system according to the present disclosure will be described below based on a first embodiment shown in the drawings. Among the mutually orthogonal X-, Y-, and Z-directions (the three axial directions) shown in the diagram, the Y-direction corresponds to the vertical direction. The Z-direction is the anteroposterior direction (working distance direction) parallel to the forward direction approaching the subject's eye (not illustrated) and the rearward direction moving away from the subject. In addition, the X-direction is the left-right direction perpendicular to both the vertical and anteroposterior directions.

### [First Embodiment]

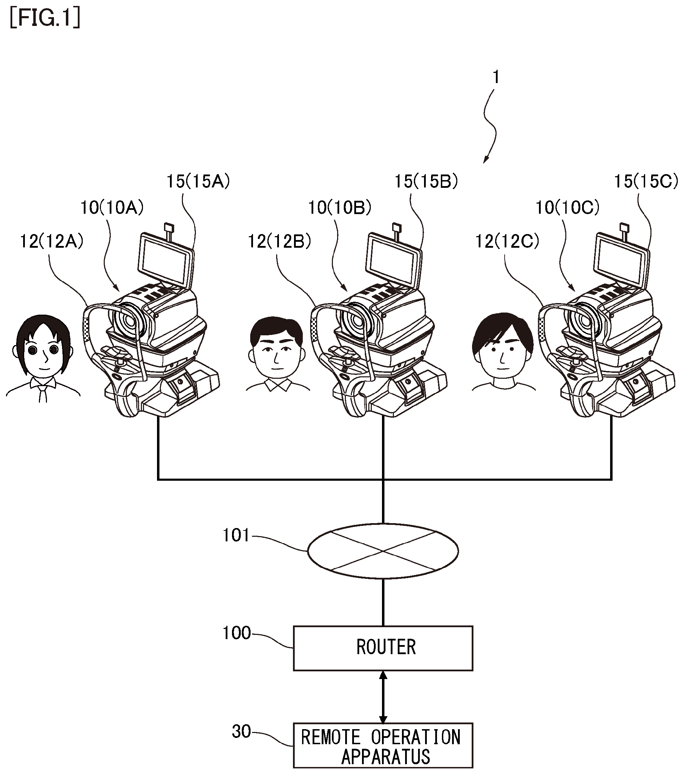

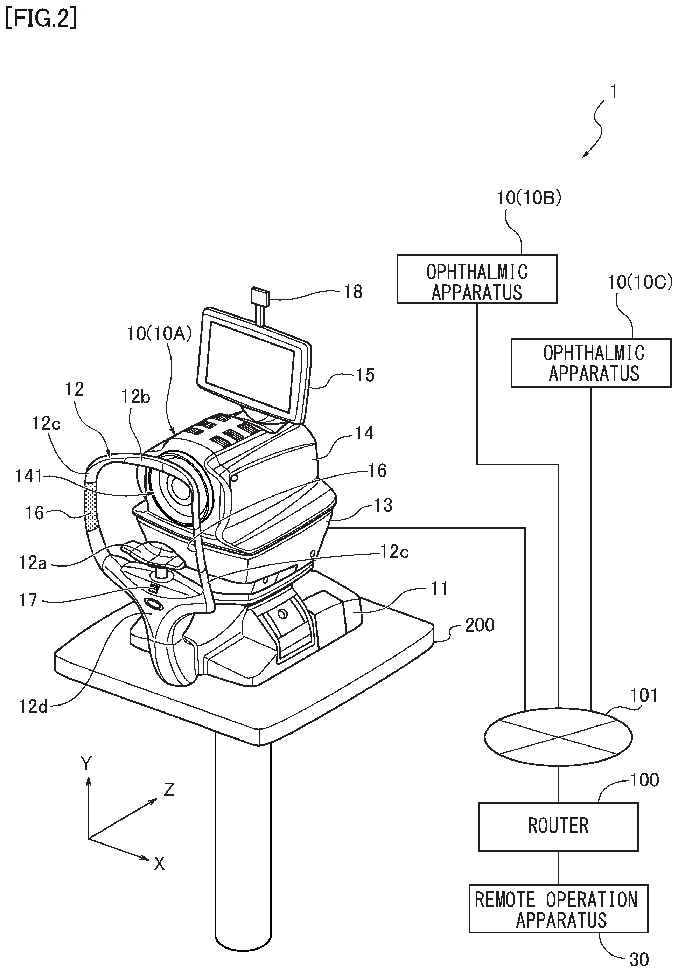

An ophthalmic system 1 according to a first embodiment will be described with reference to FIGS. 1 and 2. The ophthalmic system 1 includes three ophthalmic apparatuses 10. The three ophthalmic apparatuses 10 are a first ophthalmic apparatus 10A, a second ophthalmic apparatus 10B, and a third ophthalmic apparatus 10C.

When no particular distinction is made for the ophthalmic apparatus 10, the reference sign "10" is used alone. Examples include a plurality of ophthalmic apparatuses 10; three ophthalmic apparatuses 10; each ophthalmic apparatus 10; one ophthalmic apparatus 10; a remainder of ophthalmic apparatuses 10; and the ophthalmic apparatus 10. When distinguishing the three ophthalmic apparatuses 10, the first ophthalmic apparatus 10A, the second ophthalmic apparatus 10B, and the third ophthalmic apparatus 10C will be designated by adding letters to the reference numeral "10". Components included in the ophthalmic apparatus 10 are designated by reference signs consisting only of numerals when no particular distinction is made. Components of each of the three ophthalmic apparatuses 10 will be designated by adding letters to the reference numeral corresponding to each component. The letter "A" designates a component of the first ophthalmic apparatus 10A, the letter "B" designates a component of the second ophthalmic apparatus 10B, and the letter "C" designates a component of the third ophthalmic apparatus 10C. In the first embodiment, the first ophthalmic apparatus 10A, the second ophthalmic apparatus 10B, and the third ophthalmic apparatus 10C are assumed to share the same configuration. However, the three ophthalmic apparatuses 10 are not limited to sharing the same configuration.

The ophthalmic system 1 includes one remote operation apparatus 30 (host apparatus). The remote operation apparatus 30 is located at a location away from the three ophthalmic apparatuses 10. In other words, the three ophthalmic apparatuses 10 and the remote operation apparatus 30 are at positions (locations) separated from one another. For example, the three ophthalmic apparatuses 10 and the remote operation apparatus 30 may be arranged in adjacent rooms, respectively. Alternatively, the remote operation apparatus 30 may be installed at a hospital where an examiner is based and the three ophthalmic apparatuses 10 may be installed at locations away from the hospital. Consequently, the examiner will be present where the remote operation apparatus 30 is installed, and each subject will be present where each ophthalmic apparatus 10 is installed. The three ophthalmic apparatuses 10 may be installed in the same room or may be installed at positions (locations) separated from one another.

The remote operation apparatus 30 is connected to a communication network such as the Internet 101 via a router 100 (router device). The remote operation apparatus 30 is connected to each ophthalmic apparatus 10 via the Internet 101. Accordingly, the examiner can perform remote operations.

As shown in FIG. 2, the ophthalmic apparatus 10 is arranged on an eye examination table 200 and is used to examine an eye to be examined (hereinafter, the eye). The ophthalmic apparatus 10 is a multifunctional instrument capable of measuring characteristics including intraocular pressure, ocular refraction, and corneal curvature of the eye. Through remote operations by the examiner, the ophthalmic apparatus 10 measures various ocular characteristics of the eye, such as intraocular pressure, ocular refraction, and corneal curvature (corneal shape). The ophthalmic apparatus 10 includes a base 11, a face support 12, a drive mechanism 13, a measurement head 14, a monitor portion 15 (display portion), subject speakers 16 (speakers), a subject microphone 17 (microphone), a subject camera 18 (camera), and an apparatus control portion 20 (control portion, refer to FIG. 2).

The base 11 supports members mounted on top of the base 11 such as the face support 12 and the drive mechanism 13.

The face support 12 supports the face of the subject. The face support 12 is arranged in front of the drive mechanism 13. The face support 12 integrally includes a chin rest 12a, a forehead rest 12b, a pair of frames 12c, and a base portion 12d. The base portion 12d is fixed to the front of the base 11. The chin rest 12a supports the subject's chin. The chin rest 12a is provided above the center of the base portion 12d. The subject's forehead abuts against the forehead rest 12b. The pair of frames 12c is provided on both sides of the base portion 12d and the forehead rest 12b is connected to upper ends of the pair of frames 12c. The subject faces the ophthalmic apparatus 10 while seated in a chair or a similar fixture (not illustrated) provided in front of the ophthalmic apparatus 10. Next, the subject rests their chin on the chin rest 12a and presses their forehead against the forehead rest 12b to undergo the examination.

The drive mechanism 13 corresponds to a relative movement mechanism of the ophthalmic apparatus 10. For example, the drive mechanism 13 is implemented by an actuator (not illustrated) such as a motor. The drive mechanism 13 is driven based on control information (control signals) from the apparatus control portion 20. The drive mechanism 13 can move the chin rest 12a up and down in the Y-direction based on the control information. The drive mechanism 13 also moves the measurement head 14 in the X-, Y-, and Z-directions relative to the base 11 based on the control information. Accordingly, the measurement head 14 can be moved in the X-, Y-, and Z-directions relative to the eye. In this manner, the height of the chin rest 12a can be adjusted and the measurement head 14 can be aligned in the X-, Y-, and Z-directions relative to the eye.

An optical system for observation and capturing images is provided inside the measurement head 14. The optical system can observe and capture various ocular characteristics of the subject. Specifically, the measurement head 14 is provided with various types of ophthalmic measurement devices including an auto ref/keratometer 141. The auto ref/keratometer 141 uses various optical systems to measure characteristics including ocular refraction and corneal curvature of the eye. The specific configuration of the auto ref/keratometer 141 is known technology and, therefore, will not be described here. The measurement head 14 may be provided with devices other than the auto ref/keratometer 141 such as a non-contact tonometer. The non-contact tonometer measures intraocular pressure in the eye in a contactless manner. In other words, the measurement head 14 can be provided with any device capable of carrying out an ophthalmic examination.

The monitor portion 15 is mounted on the rear side of the upper surface of the measurement head 14. For example, the monitor portion 15 is arranged at a position that can be viewed by the subject. In other words, the monitor portion 15 is oriented (angled) in a direction of the subject (a direction in which the subject can view displays on the monitor portion 15) and enables the examiner to display various kinds of information to the subject. For example, the monitor portion 15 is oriented toward the front of the ophthalmic apparatus 10. The monitor portion 15 is also oriented in a direction in which the subject can view displays on the monitor portion 15 both when standing and when sitting in front of the ophthalmic apparatus 10.

The monitor portion 15 is, for example, a touch screen monitor, and a liquid crystal display is used. The monitor portion 15 displays images including observation images of the eye based on control information from the apparatus control portion 20. The monitor portion 15 displays measurement results of ocular characteristics including intraocular pressure, ocular refraction, and corneal curvature of the eye. The monitor portion 15 displays screens such as operation menu screens for performing various operations. The monitor portion 15 also accepts input of operations to each operation menu screen. This allows the subject to operate the ophthalmic apparatus 10 directly.

The subject speakers 16 are built into the pair of frames 12c, respectively. The subject speakers 16 are positioned near the left and right ears of the subject when the subject's eye is being examined.

The subject microphone 17 is built into a central part of the base portion 12d and in front of and below the chin rest 12a. The subject microphone 17 is positioned in a vicinity of the mouth of the subject when the subject's eye is being examined.

The subject camera 18 is provided above the monitor portion 15. The subject camera 18 is positioned so as to capture the subject in an examination posture. Thus, the subject camera 18 is capable of capturing images of the subject in the examination posture as well as of the subject standing in front of the face support 12. In addition, the subject camera 18 is a peripheral camera capable of capturing images of the area surrounding the ophthalmic apparatus 10, specifically the area in front of the subject camera 18. Images of the subject standing in front of the face support 12 are captured as images of the subject arriving for an examination. Images of the area surrounding the ophthalmic apparatus 10 are captured as images of the subject not appearing in front of the face support 12.

Examination postures include a posture in which the subject is seated in a chair or a similar fixture (not illustrated) provided in front of the ophthalmic apparatus 10, a posture in which the subject rests their chin on the chin rest 12a and their forehead against the forehead rest 12b, and a posture in which the subject is standing in front of the face support 12. As described later, the examiner can confirm subject display information (captured images, moving images, video, and (video) display information) of the subject camera 18 when the subject display information is displayed on an examiner monitor portion 32. Thus, the examiner can observe the subject's state before, during, and after the examination via the display on the examiner monitor portion 32. The examiner can also use the display on the examiner monitor portion 32 to adjust the position of the ophthalmic apparatus 10 relative to the subject.

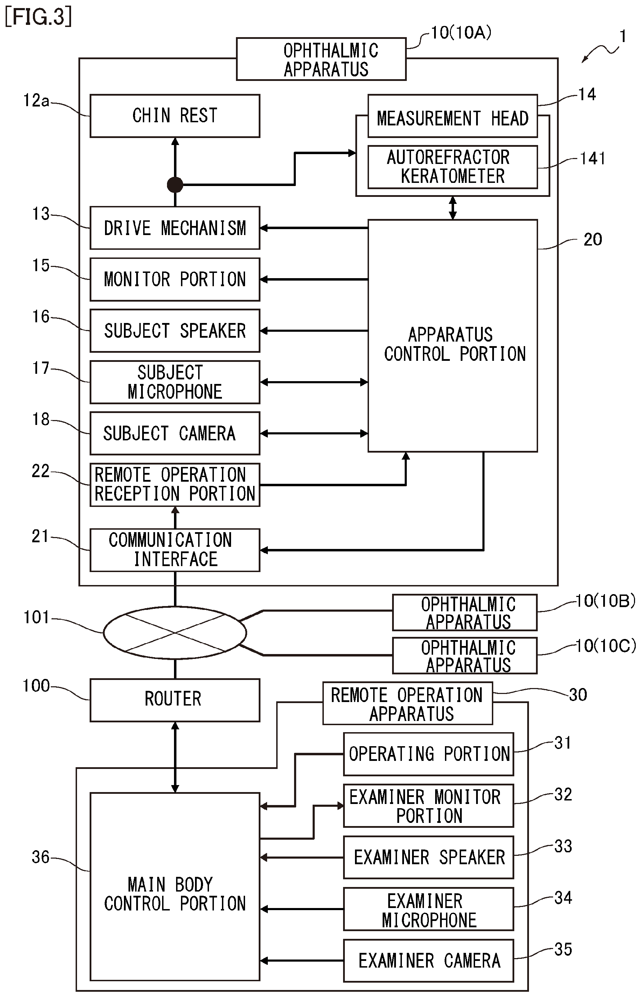

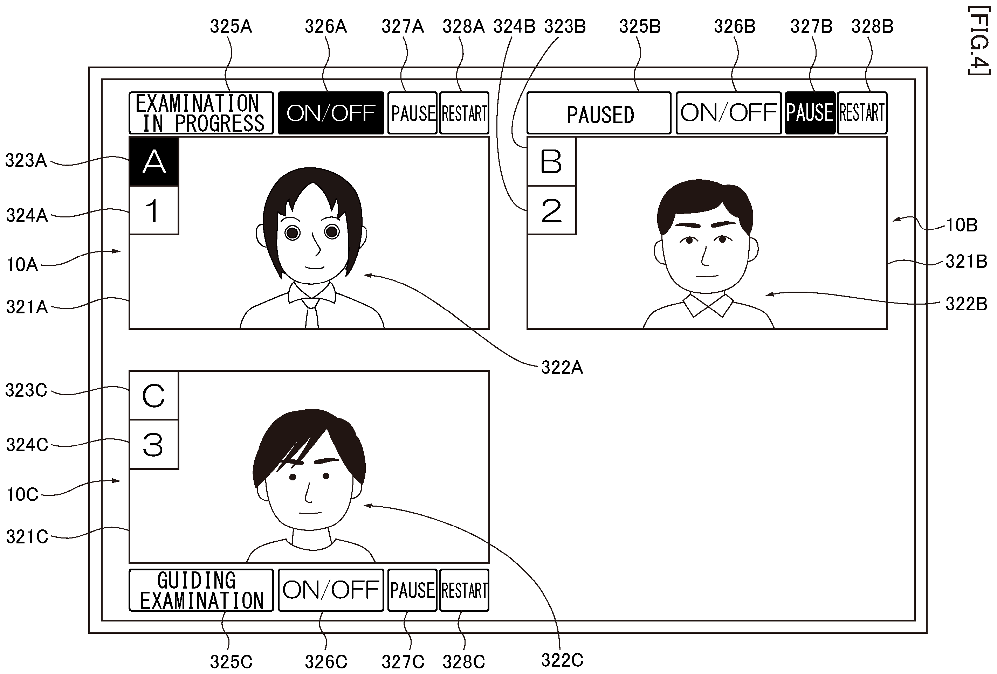

Next, a control system of the ophthalmic system 1 will be described below with reference to FIGS. 3 and 4.

In addition to the components described above, the ophthalmic apparatus 10 includes the apparatus control portion 20, a communication interface 21, and a remote operation reception portion 22 as shown in FIG. 3.

The remote operation apparatus 30 includes an operating portion 31, the examiner monitor portion 32, an examiner speaker 33 (speaker), an examiner microphone 34 (microphone), an examiner camera 35 (camera), and a main body control portion 36.

The communication interface 21 and the main body control portion 36 are connected to one another via the Internet 101 and the router 100. The communication interface 21 and the main body control portion 36 exchange various types of information (such as control signals) with each other via the router 100 and the Internet 101. Hereinafter, the description "via the router 100 and the Internet 101" may be omitted.

Given that the ophthalmic apparatus 10 is remotely operated by the examiner, the remote operation apparatus 30 will be described first.

The remote operation apparatus 30 is constituted by, for example, a personal computer or a tablet terminal. The remote operation apparatus 30 is used when the examiner remotely operates the ophthalmic apparatus 10.

The operating portion 31 is constituted by, for example, a keyboard and a mouse. The operating portion 31 receives operation information for the examiner to remotely operate the ophthalmic apparatus 10. The operating portion 31 outputs the received operation information to the main body control portion 36. In addition to input by the examiner, the operation information includes information (signals) automatically output by the main body control portion 36 to the remote operation reception portion 22 based on various types of information including information of the ophthalmic apparatus 10.

The examiner monitor portion 32 is constituted by, for example, a liquid crystal display. The examiner monitor portion 32 displays display information received from the main body control portion 36. The display information includes display information of an examination mode and a sleep mode (to be described later) of the ophthalmic apparatus 10, the subject display information of the subject camera 18, and the same display information as the display screens on the monitor portion 15 of the ophthalmic apparatus 10.

FIG. 4 illustrates an example of a display on the examiner monitor portion 32. The examiner monitor portion 32 displays the subject and other information. The display on the examiner monitor portion 32 such as that shown in FIG. 4 allows the examiner to recognize the state (status) of each ophthalmic apparatus 10 and each subject. The examiner monitor portion 32 displays an image 321 separately for each of the three ophthalmic apparatuses 10. The image 321 of each ophthalmic apparatus 10 displays a subject photograph 322, an ophthalmic apparatus symbol 323, an examination sequence number 324, an ophthalmic examination status 325, a function switch 326 (output portion), a pause switch 327 (output portion), and a restart switch 328 (output portion).

The subject photograph 322 is a photograph of the subject captured by the subject camera 18. The photograph is a still image of the face of the subject captured for identity verification. The still image may be changed every few seconds, or may remain unchanged if the purpose is to recognize the subject. A moving image of the subject may be used as the subject photograph 322 instead of a still image.

The ophthalmic apparatus symbol 323 is a symbol denoting each ophthalmic apparatus 10. "A" denotes the first ophthalmic apparatus 10A, "B" denotes the second ophthalmic apparatus 10B, and "C" denotes the third ophthalmic apparatus 10C.

The examination sequence number 324 indicates the order in which the subjects are ready for examination.

The ophthalmic examination status 325 indicates the status of the ophthalmic examination at each ophthalmic apparatus 10. "Examination in Progress" indicates that the subject's ophthalmic examination is actually being performed. "Paused" indicates that an interactive function (to be described later) is paused. "Guiding examination" indicates that examination guidance processing (to be described later) is being performed. Other statuses include "Absent" indicating that the subject is not present, "Standing By" indicating that the subject's ophthalmic examination is ready and the subject is awaiting its start, and "Examination Complete" indicating that the examiner has completed the ophthalmic examination.

The function switch 326 indicates whether the interactive function (to be described later) is enabled or disabled and also serves as a switch to enable or disable the interactive function. White letters against a black background indicate that the interactive function is enabled, and black letters against a white background indicate that the interactive function is disabled.

The pause switch 327 indicates whether or not the interactive function has been paused from an enabled state and also serves as a switch to pause the enabled interactive function. White letters against a black background indicate that the interactive function is paused, and black letters against a white background indicate that the interactive function is not paused.

The restart switch 328 indicates whether or not the interactive function has been restarted from a paused state and also serves as a switch to restart the paused interactive function. White letters against a black background indicate that the interactive function is restarted, and black letters against a white background indicate that the interactive function is not restarted.

The examiner speaker 33 and the examiner microphone 34 shown in FIG. 3 are constituted by, for example, a headset (headphone with a microphone). The examiner speaker 33 outputs audio of the subject (subject audio information) input to the subject microphone 17. The examiner microphone 34 outputs received audio of the examiner (examiner audio information) to the main body control portion 36.

The examiner camera 35 is provided at a position where the examiner can be captured when the examiner is at least positioned where the examiner can operate the remote operation apparatus 30. As described later, both the subject and the examiner can confirm examiner display information (captured images, moving images, video, and (video) display information) of the examiner camera 35 when the examiner display information is displayed on the monitor portion 15 and the examiner monitor portion 32. As a result, the subject can recognize the presence of the examiner via the display of the monitor portion 15. The examiner can also recognize how the examiner display information is displayed on the monitor portion 15 via a display on the examiner monitor portion 32. "When the examiner is positioned where the remote operation apparatus 30 can be operated" also includes during remote operation by the examiner.

The main body control portion 36 includes a processing circuit constituted by components including various processors and memories (storage portions). The various processors include central processing portions (CPUs), graphics processing portions (GPUs), application-specific integrated circuits (ASICs), and programmable logic devices [such as simple programmable logic devices (SPLDs), complex programmable logic devices (CPLDs), and field-programmable gate arrays (FPGAs)]. The various controls (functions) of the main body control portion 36 may be realized by one processor or a plurality of similar or different processors.

The main body control portion 36 outputs information including operation information, examiner audio information, and examiner display information to the communication interface 21. The main body control portion 36 receives information including display information and subject audio information from the communication interface 21. The main body control portion 36 controls operations of the remote operation apparatus 30 by receiving information such as display information and subject audio information and reading and executing a control program stored in a memory (not illustrated). For example, the main body control portion 36 outputs display information to the examiner monitor portion 32 and outputs subject audio information to the examiner speaker 33. In addition, based on information such as operation information received from the operating portion 31, the main body control portion 36 outputs information such as the operation information to the apparatus control portion 20. The main body control portion 36 communicates with each ophthalmic apparatus 10 and stores a status (information) of each ophthalmic apparatus 10 in a memory. Examples of the status of each ophthalmic apparatus 10 include a status of the interactive function (to be described later) including enabled, disabled, paused (temporarily suspended), and restarted. Furthermore, the main body control portion 36 stores, in a memory, the order in which the interactive function of each ophthalmic apparatus 10 was enabled (when "enable" control was performed in Step S52 described later). As described above, the main body control portion 36 integrally manages the status of each ophthalmic apparatus 10.

Next, the ophthalmic apparatus 10 will be described.

The communication interface 21 and the remote operation reception portion 22 are implemented by a local area network (LAN) chip. The communication interface 21 receives information including operation information, examiner audio information, and examiner display information from the main body control portion 36. The communication interface 21 outputs information including operation information, examiner audio information, and examiner display information to the remote operation reception portion 22. The communication interface 21 also receives information including display information, subject audio information, and the status of each ophthalmic apparatus 10 from the apparatus control portion 20. The communication interface 21 outputs information including display information, subject audio information, and the status of each ophthalmic apparatus 10 to the main body control portion 36. The LAN chip is an example of a component that, while operating in the sleep mode (to be described later), receives signals to start (restart) a part of the ophthalmic apparatus 10 having been inactive.

The remote operation reception portion 22 receives information including operation information, examiner audio information, and examiner display information from the communication interface 21. The remote operation reception portion 22 receives information including operation information, examiner audio information, and examiner display information and outputs the information to the apparatus control portion 20. The remote operation reception portion 22 receives a wake signal or a sleep signal as the operation information and outputs the signal to the apparatus control portion 20. The remote operation reception portion 22 receives an enable signal, a disable signal, a pause signal, and a restart signal of the interactive function (to be described later) and outputs the signals to the apparatus control portion 20.

The apparatus control portion 20 includes a processing circuit constituted by components including various processors and memories in a similar manner to the main body control portion 36 described above. Various controls (functions) of the apparatus control portion 20 may be realized by one processor or a plurality of similar or different processors.

The apparatus control portion 20 controls operations of the ophthalmic apparatus 10 by reading and executing a control program stored in a memory (not illustrated) based on information including operation information, examiner audio information, and examiner display information received from the remote operation reception portion 22. The apparatus control portion 20 outputs information such as subject audio information to the main body control portion 36 based on information such as subject audio received from the subject microphone 17. The apparatus control portion 20 functions to control mode transitions, examination guidance, examination execution, display, and audio. The apparatus control portion 20 also stores the status of the ophthalmic apparatus 10 in a memory.

Upon receiving a sleep signal from the remote operation reception portion 22, the apparatus control portion 20 controls the ophthalmic apparatus 10 to disable power supply to all components with the exception of the communication interface 21 and the remote operation reception portion 22. The apparatus control portion 20 suspends the operation of the ophthalmic apparatus 10 with the exception of some components, and causes the ophthalmic apparatus 10 to transition to the sleep mode. Alternatively, upon receiving a wake signal from the remote operation reception portion 22, the apparatus control portion 20 controls the ophthalmic apparatus 10 to enable power supply to all components, and causes the ophthalmic apparatus 10 to transition to the examination mode.

The sleep mode is a mode in which operations of a part of the ophthalmic apparatus 10 are suspended, while a component that receives signals for activating (restarting) the suspended part of the ophthalmic apparatus 10 remains in operation. In other words, the sleep mode is a mode that puts the ophthalmic apparatus 10 in a state where operations are suspended and the ophthalmic apparatus 10 awaits a start signal (a suspended state or a standby state). The sleep mode is also a mode that enables power consumption by the ophthalmic apparatus 10 to be reduced compared to the examination mode due to the suspension of operations of a part of the ophthalmic apparatus 10 (low-power consumption state), and the ophthalmic apparatus 10 can switch to the examination mode more quickly than when power is completely turned off. The examination mode is a mode (started state) in which examinations can be performed by the ophthalmic apparatus 10.

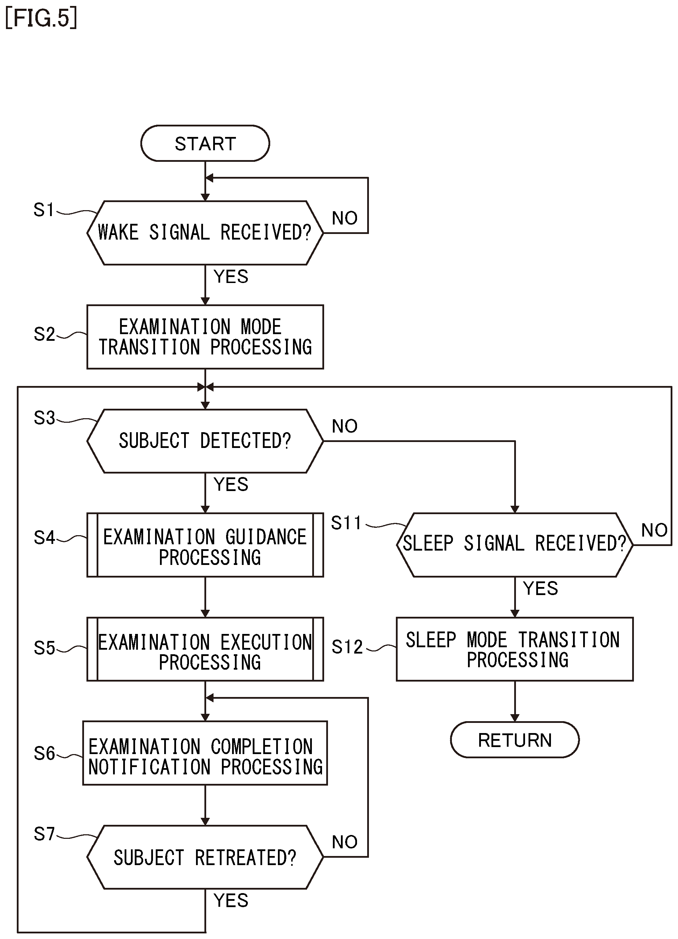

Next, a flow of processing by the ophthalmic apparatus 10 will be described below using the exemplary flow charts in FIGS. 5 to 9. In the flow charts shown in FIGS. 5 to 9, the various kinds of information output from the main body control portion 36 are input to the apparatus control portion 20 via the communication interface 21 and the remote operation reception portion 22. Thus, the descriptions of "via the communication interface 21" and "via the communication interface 21 and the remote operation reception portion 22" may be omitted when describing the flow charts. Conversely, the various kinds of information output from the apparatus control portion 20 are input to the main body control portion 36 via the communication interface 21. Thus, the descriptions of "via the communication interface 21" may be omitted.

First, the flow of processing by the ophthalmic apparatus 10 will be described below using the exemplary flow chart in FIG. 5. Given that the ophthalmic apparatus 10 is in the sleep mode until the examiner begins remote operation, the program starts the flow of processing in the sleep mode. "Until the examiner begins remote operation" means, for example, until hospital appointments begin.

In the sleep mode of the ophthalmic apparatus 10 according to the first embodiment, operations of the chin rest 12a, the drive mechanism 13, the measurement head 14, the monitor portion 15, the subject speakers 16, the subject camera 18, and the apparatus control portion 20 are suspended. The communication interface 21 and the remote operation reception portion 22 remain in operation even in the sleep mode. The communication interface 21 and the remote operation reception portion 22 are components that receive signals for starting (restarting) the ophthalmic apparatus 10.

In Step S1, following the start of processing, the remote operation reception portion 22 determines whether or not a wake signal has been received. When a determination of YES is made, the processing proceeds to Step S2, but when a determination of NO is made, Step S1 is repeated. When in the sleep mode, the ophthalmic apparatus 10 awaits a wake signal from the main body control portion 36. Once a wake signal is output from the main body control portion 36 in response to an operation input by the examiner to the operating portion 31, the remote operation reception portion 22 receives the wake signal. The remote operation reception portion 22 determines that a wake signal has been received. If a determination that a wake signal has not been received continues, Step S1 is repeated.

In Step S2, following the reception of the wake signal in Step S1, the remote operation reception portion 22 and the apparatus control portion 20 execute examination mode transition processing and the processing proceeds to Step S3. In the examination mode transition processing, the remote operation reception portion 22 outputs, to the apparatus control portion 20, the wake signal received in Step S1. The apparatus control portion 20 starts (operates) upon receiving the wake signal. Next, the apparatus control portion 20 starts the chin rest 12a, the drive mechanism 13, the measurement head 14, the monitor portion 15, the subject speakers 16, and the subject camera 18. In other words, the apparatus control portion 20 turns on all power supplies to the ophthalmic apparatus 10.

The apparatus control portion 20 does not start the subject microphone 17 even if a wake signal is received. Next, the apparatus control portion 20 performs mode transition control to switch the ophthalmic apparatus 10 from sleep mode to examination mode. After performing the mode transition control to switch the ophthalmic apparatus 10 from sleep mode to examination mode, the apparatus control portion 20 may start the suspended part of the ophthalmic apparatus 10 (such as the chin rest 12a).

In Step S3, following the examination mode transition processing in Step S2, a determination of a retreat detection in Step S7, or a determination of non-reception of a sleep signal in Step S11, the apparatus control portion 20 determines whether or not the subject has been detected. When a determination of YES is made, the processing proceeds to Step S4, but when a determination of NO is made, the processing proceeds to Step S11. The detection of the subject or, in other words, the subject presence detection is determined based on a moving image (video) taken by the subject camera 18. First, the subject display information of the subject camera 18 is output from the subject camera 18 to the main body control portion 36 via the apparatus control portion 20. As a result, the main body control portion 36 displays the subject display information on the examiner monitor portion 32 separately from the display shown in FIG. 4. If FIG. 4 is a moving image, the subject display information may be combined with the display in FIG. 4. The main body control portion 36 displays the subject display information on the examiner monitor portion 32 when the subject camera 18 is in operation. Still images captured every few seconds may be displayed instead of a moving image, or both a moving image and still images may be displayed on the examiner monitor portion 32.

Next, the examiner confirms the presence of the subject based on the display on the examiner monitor portion 32. The presence of the subject is not limited to the subject's entire body being visible and also includes only a part of the subject such as the head being visible. As a result of the recognition of the presence of the subject, presence detection information or presence non-detection information is output as operation information from the main body control portion 36 to the apparatus control portion 20 in response to an operation input by the examiner to the operating portion 31. Presence detection information refers to information indicating that the examiner has recognized the presence of the subject. Presence non-detection information refers to information indicating that the examiner has not recognized the presence of the subject. When the presence detection information is received, the apparatus control portion 20 determines that the presence has been detected in Step S3. On the other hand, when the presence non-detection information is received, the apparatus control portion 20 determines that the presence has not been detected in Step S3. The confirmation of the presence of the subject may be automatically performed by an artificial intelligence (AI) processing portion instead of the examiner. To this end, the artificial intelligence processing portion is to be installed in the remote operation apparatus 30 together with an AI model and other components.

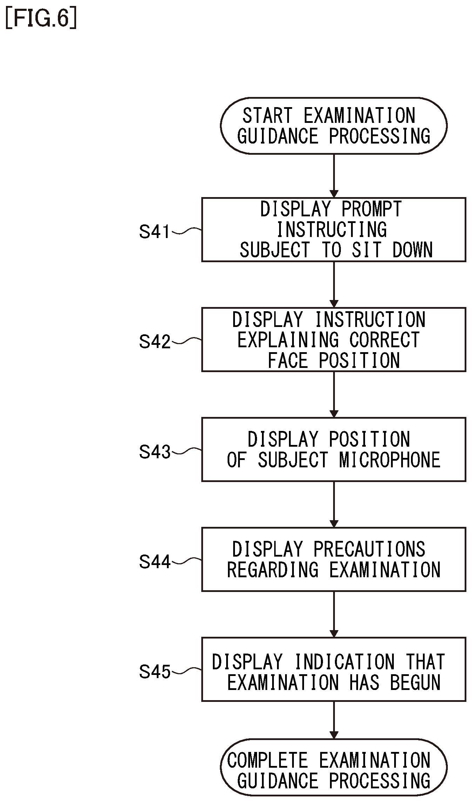

In Step S4, following the determination that the presence is detected in Step S3, the apparatus control portion 20 executes examination guidance processing and the processing proceeds to Step S5. The examination guidance processing will be described later with reference to the flow chart in FIG. 6.

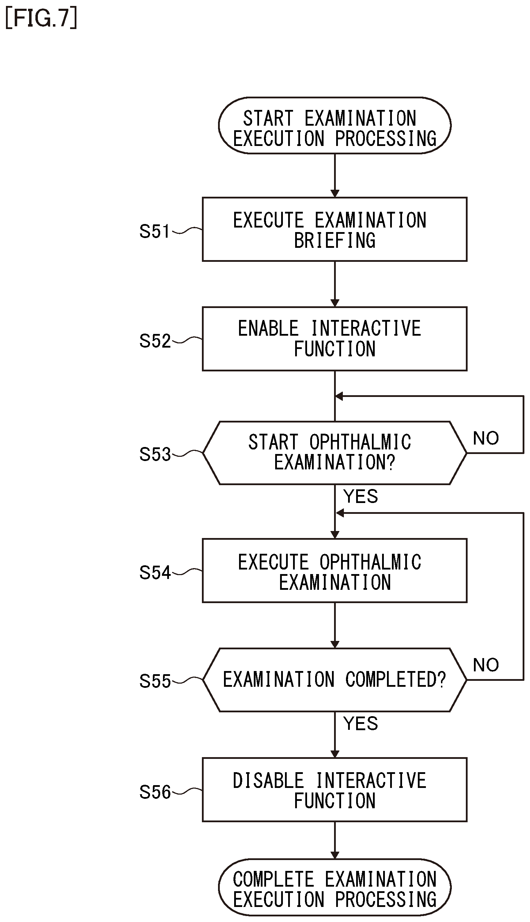

In Step S5, following the examination guidance processing in Step S4, the apparatus control portion 20 performs examination execution processing and the processing proceeds to Step S6. The examination execution processing will be described later with reference to the flow chart in FIG. 7.

In Step S6, following the examination execution processing in Step S5 or the determination of a retreat non-detection in Step S7, the apparatus control portion 20 executes examination completion notification processing and the processing proceeds to Step S7. The examination completion notification processing is processing of notifying the subject that the examination has been completed. In the present processing, when the processing proceeds to Step S6, the subject speakers 16 automatically output automated audio information in response to automatically receiving the information from the apparatus control portion 20. Alternatively, when the monitor portion 15 receives completion notification display information notifying completion from the apparatus control portion 20, the monitor portion 15 may display the information. Examples of the automated audio information and the completion notification display information include information such as "Please remove your face from the frame. All examinations have been completed. Thank you for your cooperation." At this point, when the processing returns to Step S6 following a determination of retreat non-detection in Step S7, the subject will be prompted to retreat once again.

As described above, once the ophthalmic examination is completed (Step S5), the apparatus control portion 20 executes examination completion notification processing of notifying the subject that the examination has been completed. In other words, because the remote operation apparatus 30 is located remotely from the ophthalmic apparatus 10, the subject needs to be notified of the completion of the examination. Executing the examination completion notification processing enables the subject to be notified that the examination has been completed and to recognize the completion of the examination.

In Step S7, following the examination completion notification processing in Step S6, the apparatus control portion 20 determines whether or not the subject has retreated. When a determination of YES is made, the processing returns to Step S3, but when a determination of NO is made, the processing returns to Step S6. The non-detection of the subject or, in other words, the subject retreat detection is determined based on a moving image taken by the subject camera 18. First, the subject display information of the subject camera 18 is output from the subject camera 18 to the main body control portion 36 via the apparatus control portion 20. As a result, the main body control portion 36 displays the subject display information on the examiner monitor portion 32. The main body control portion 36 displays the subject display information on the examiner monitor portion 32 when the subject camera 18 is in operation.

Next, the examiner confirms the retreat of the subject based on the display on the examiner monitor portion 32. The subject is determined to have retreated when the subject's entire body is not visible and not when a part of the subject such as the head is visible. As a result of the recognition of the retreat of the subject, retreat detection information or retreat non-detection information is output as operation information from the main body control portion 36 to the apparatus control portion 20 in response to an operation input by the examiner to the operating portion 31. Retreat detection information refers to information indicating that the examiner has not recognized the presence of the subject but has recognized a retreat by the subject. Retreat non-detection information refers to information indicating that the examiner has recognized the presence of the subject but has not recognized a retreat by the subject. When the retreat detection information is received, the apparatus control portion 20 determines that a retreat has been detected in Step S7. On the other hand, when the retreat non-detection information is received, the apparatus control portion 20 determines that a retreat has not been detected in Step S7. At this point, if a determination that a retreat has not been detected continues, Steps S6 and S7 are repeated. As described in relation to Step S3, the confirmation of the retreat of the subject may be automatically performed by an artificial intelligence (AI) processing portion instead of the examiner.

In Step S11, following the determination of presence non-detection in Step S3, the remote operation reception portion 22 determines whether or not a sleep signal has been received. When a determination of YES is made, the processing proceeds to Step S12, but when a determination of NO is made, the processing returns to Step S3. In Step S11, when the ophthalmic apparatus 10 is in examination mode, the remote operation reception portion 22 receives a sleep signal after the sleep signal is output from the main body control portion 36 in response to an operation input by the examiner to the operating portion 31. The remote operation reception portion 22 determines that a sleep signal has been received. At this point, if the determination that the presence is not detected in Step S3 and the determination that a sleep signal has not been received in Step S11 continue, Steps S3 and S11 are repeated.

In Step S12, following the reception of the sleep signal in Step S11, the remote operation reception portion 22 and the apparatus control portion 20 execute sleep mode transition processing and proceed to return. In the sleep mode transition processing, the remote operation reception portion 22 outputs, to the apparatus control portion 20, the sleep signal received in Step S11. Accordingly, the apparatus control portion 20 suspends operations of the chin rest 12a, the drive mechanism 13, the measurement head 14, the monitor portion 15, and the subject camera 18.

When the subject speakers 16 and the subject microphone 17 are in operation, the apparatus control portion 20 suspends operations of the subject speakers 16 and the subject microphone 17. Next, the apparatus control portion 20 performs mode transition control to switch the ophthalmic apparatus 10 from examination mode to sleep mode and suspends operations of the apparatus control portion 20. In other words, the apparatus control portion 20 controls the ophthalmic apparatus 10 to disable power supply to all components with the exception of the communication interface 21 and the remote operation reception portion 22.

As described above, when a sleep signal is received from the remote operation apparatus 30 (YES in Step S11), the apparatus control portion 20 causes the ophthalmic apparatus 10 to transition from examination mode to sleep mode (Step S12, return). However, the apparatus control portion 20 operates on the condition that the subject camera 18 has not detected the subject (NO in Step S3). In other words, the examiner need not visit a remote location to cause a transition from examination mode to sleep mode. As a result, when the examiner remotely operates the ophthalmic apparatus 10 using the remote operation apparatus 30, the examiner can remotely cause a transition from sleep mode to examination mode. Considering that operating the ophthalmic apparatus 10 results in wasted power consumption if the subject is not detected, the ophthalmic apparatus 10 makes a transition from examination mode to sleep mode by remote operation by the examiner. As a result, the power consumption of the ophthalmic apparatus 10 can be reduced compared to the examination mode.

Next, a flow of the examination guidance processing in Step S4 will be described below using the exemplary flow chart in FIG. 6.

Steps S41 through S45 constitute the examination guidance processing for guiding the subject to the examination (ophthalmic apparatus 10). The apparatus control portion 20 controls examination guidance control for the examination guidance processing. For example, when the processing proceeds to Step S4, the monitor portion 15 displays guidance display information in response to automatically receiving the information from the apparatus control portion 20 (guidance control). The guidance display information is information for guiding the subject to the ophthalmic apparatus 10.

In Step S41, following the start of the examination guidance processing, the apparatus control portion 20 displays a prompt on the monitor portion 15 instructing the subject to sit down and the processing proceeds to Step S42.

In Step S42, following the display in Step S41, the apparatus control portion 20 displays an instruction explaining a correct position for the subject's face on the monitor portion 15 and the processing proceeds to Step S43.

In Step S43, following the display in Step S42, the apparatus control portion 20 displays the position of the subject microphone 17 on the monitor portion 15 and the processing proceeds to Step S44.

In Step S44, following the display in Step S43, the apparatus control portion 20 displays precautions regarding the examination by the ophthalmic apparatus 10 on the monitor portion 15 and the processing proceeds to Step S45.

In Step S45, following the display in Step S44, the apparatus control portion 20 displays an indication that the examination by the ophthalmic apparatus 10 has begun on the monitor portion 15 and the examination guidance processing is to be completed.

Next, a flow of the examination execution processing in Step S5 will be described below using the exemplary flow chart in FIG. 7.

Steps S51 through S56 constitute the examination execution processing in which the eye is examined by the ophthalmic apparatus 10. The examination execution processing is controlled by the apparatus control portion 20 and remote operations by the examiner.

In Step S51, following the start of the examination execution processing, the apparatus control portion 20 executes examination briefing and the processing proceeds to Step S52. The execution of examination briefing involves explaining that the examination is about to begin and explaining that the subject can communicate with the examiner by speaking into the subject microphone 17 if they have any questions or concerns. The examination briefing is executed when the processing proceeds to Step S51, and the subject speakers 16 automatically output automated audio information in response to automatically receiving the automated audio information from the apparatus control portion 20. Examples of the automated audio information include statements such as "We are now going to perform a refraction examination. If you have any questions or concerns, please speak into the microphone to communicate with us."

In Step S52, following the execution of the examination briefing in Step S51, the apparatus control portion 20 enables the interactive function and the processing proceeds to Step S53. Enabling the interactive function involves automatically outputting an enable signal that turns on the subject microphone 17 from the main body control portion 36 to the apparatus control portion 20. Accordingly, the apparatus control portion 20 performs enable control (audio control) of switching the subject microphone 17 from off to on. The subject audio received by the subject microphone 17 is output to the main body control portion 36 via the apparatus control portion 20 until the subject microphone 17 is switched off. Accordingly, the main body control portion 36 can convey questions and other remarks of the subject to the examiner by outputting subject audio information from the examiner speaker 33. The interactive function allows the subject and the examiner to communicate with each other when enabled.

In Step S53, following the enable control of the interactive function in Step S52, the apparatus control portion 20 determines whether or not to start the ophthalmic examination. When a determination of YES is made, the processing proceeds to Step S54, but when a determination of NO is made, Step S53 is repeated. An execution start signal of the ophthalmic examination is output to the apparatus control portion 20 via the main body control portion 36 in response to an operation input by the examiner to the operating portion 31. Accordingly, execution of the ophthalmic examination is actually started by the examiner. If an execution start signal of the ophthalmic examination is not output to the apparatus control portion 20, Step S53 is repeated. In other words, the subject stands by, awaiting the start of the ophthalmic examination.

In Step S54, following the start of execution of the ophthalmic examination in Step S53 or a determination that the examination has not been completed in Step S55, the ophthalmic examination is actually executed by the examiner and the processing proceeds to Step S55. The ophthalmic examination is executed by the examiner speaking into the examiner microphone 34 and inputting operations to the operating portion 31. Examiner audio input to the examiner microphone 34 and operation information are output to the apparatus control portion 20 via the main body control portion 36. Accordingly, the apparatus control portion 20 enables the examiner to issue instructions to the subject by outputting examiner audio information from the subject speakers 16 (audio control).

The apparatus control portion 20 also drives the drive mechanism 13 based on the operation information to adjust a position of the chin rest 12a in the Y-direction and approximately align the auto ref/keratometer 141 in the X, Y, and Z directions relative to the eye. In addition, the apparatus control portion 20 controls the execution of examinations necessary for an ophthalmic examination based on the operation information. Information on the measurement result by the auto ref/keratometer 141 is displayed on the monitor portion 15 from the measurement head 14 via the apparatus control portion 20. The measurement result information received by the apparatus control portion 20 is displayed on the examiner monitor portion 32 via the main body control portion 36.

In Step S55, following the execution of the ophthalmic examination in Step S54, the apparatus control portion 20 determines whether or not the ophthalmic examination has actually been completed. When a determination of YES is made, the processing proceeds to Step S56, but when a determination of NO is made, the processing returns to Step S54. The ophthalmic examination is completed when all examination items set in advance have been completed. For this reason, the apparatus control portion 20 determines whether or not all examination items set in advance have been completed based on the operation information and the measurement result information.

As described above, first, in Step S52, the apparatus control portion 20 enables the interactive function that allows the subject and the examiner to communicate with each other while in examination mode and an examination is in progress. Next, the examiner executes an ophthalmic examination by the ophthalmic apparatus 10 (Step S54 and NO in Step S55 in FIG. 7). As a result, the examiner and the subject can talk to each other during an examination period in which the ophthalmic examination is actually being executed by the ophthalmic apparatus 10.

In Step S56, following the determination of examination completion in Step S55, the apparatus control portion 20 disables the interactive function and the processing proceeds to complete the examination execution processing. Disabling the interactive function involves automatically outputting a disable signal that turns off the subject microphone 17 from the main body control portion 36 to the apparatus control portion 20. Accordingly, the apparatus control portion 20 performs disable control (audio control) of switching the subject microphone 17 from on to off.

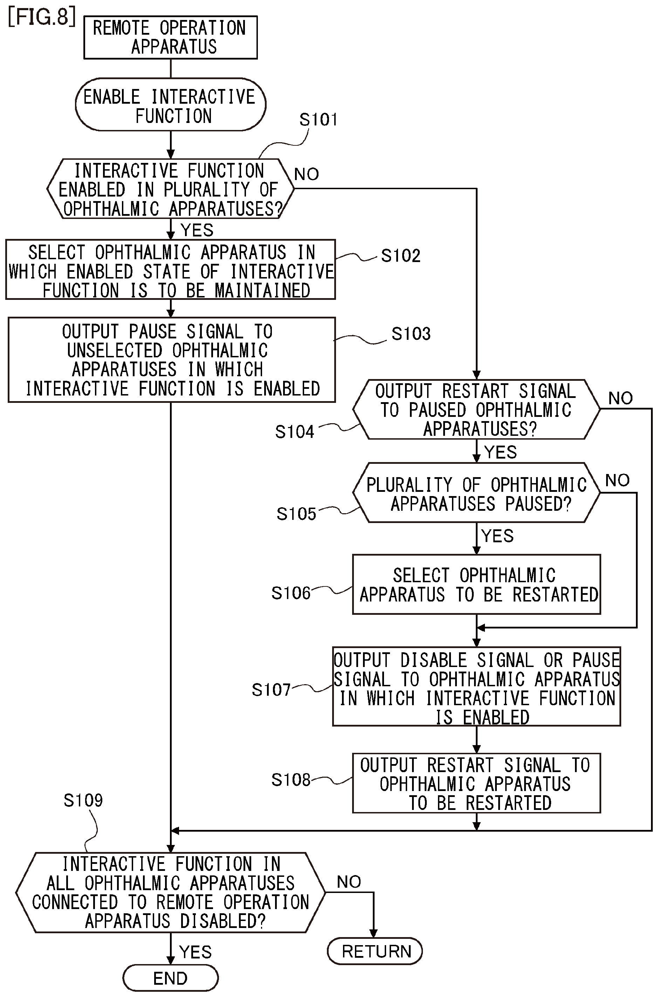

Next, when the interactive function is enabled, FIG. 8 illustrates an example flow of execution performed by the remote operation apparatus 30, as described below. The flow chart in FIG. 8 represents processing executed separately from FIGS. 3 and 5 and FIG. 9 (to be described later) when enable control of the interactive function in Step S52 is executed in at least one of the three ophthalmic apparatuses 10. Steps S101 through S109 constitute processing related to the interactive function which is performed by the remote operation apparatus 30 (main body control portion 36).

In at least one of the three ophthalmic apparatuses 10, the apparatus control portion 20 executes enable control of the interactive function in Step S52 in response to the main body control portion 36 outputting an enable signal. Accordingly, the flow chart in FIG. 8 starts processing at Step S101.

In Step S101, following enable control processing of the interactive function in Step S52 or return, the main body control portion 36 determines whether or not the interactive function is enabled in a plurality of (two or more) ophthalmic apparatuses 10. When a determination of YES is made, the processing proceeds to Step S102, but when a determination of NO is made, the processing proceeds to Step S104.

The enable and disable controls of the interactive function in each ophthalmic apparatus 10 are as described earlier in Steps S52 and S56. Specifically, the main body control portion 36 automatically outputs an enable signal or a disable signal to the apparatus control portion 20, thereby enabling or disabling the interactive function. As described earlier, the main body control portion 36 stores any of the states of enabled, disabled, paused, and restarted of the interactive function of each ophthalmic apparatus 10 in a memory (not illustrated). For this reason, the main body control portion 36 can determine whether or not the interactive function is enabled or disabled in each of the plurality of ophthalmic apparatuses 10.

A determination of NO in Step S101 means that enable control of the interactive function is being executed by the apparatus control portion 20 in only one of the three ophthalmic apparatuses 10. An example would be a situation where the interactive function is enabled in the first ophthalmic apparatus 10A but disabled or paused in the second ophthalmic apparatus 10B and the third ophthalmic apparatus 10C, which are the remainder of the ophthalmic apparatuses. In this situation, enable control is maintained in the first ophthalmic apparatus 10A.

In Step S102, following the determination of YES in Step S101, the main body control portion 36 selects one ophthalmic apparatus 10 in which the enabled state of the interactive function is to be maintained among the plurality of ophthalmic apparatuses 10 whose interactive function is enabled, and the processing proceeds to Step S103.

For example, the main body control portion 36 selects a subject who is ready for examination from among the plurality of ophthalmic apparatuses 10. Specifically, the main body control portion 36 makes the selection based on the order of the ophthalmic apparatuses 10 in which the subjects started waiting. In other words, the main body control portion 36 selects, from the plurality of ophthalmic apparatuses 10, the ophthalmic apparatus 10 for which the interactive function was first enabled.

When the interactive function is enabled (enable control in Step S52) in a plurality of ophthalmic apparatuses 10, the main body control portion 36 selects a single ophthalmic apparatus 10 that was the first to have its interactive function enabled from among the plurality of ophthalmic apparatuses 10. The selected ophthalmic apparatus 10 is the one that starts the ophthalmic examination. As described earlier, the order in which the interactive function was enabled is stored in the memory of the main body control portion 36.

In Step S103, following the selection of the single ophthalmic apparatus 10 in Step S102, the main body control portion 36 outputs a pause signal to the unselected ophthalmic apparatuses 10 and the processing proceeds to Step S109. The pause signal is a signal that pauses the interactive function in the ophthalmic apparatus 10 or, in other words, a signal that temporarily switches the interactive function from on to off. The main body control portion 36 outputs the pause signal by controlling the pause switch 327. For example, when the first ophthalmic apparatus 10A is selected in Step S102, the main body control portion 36 outputs the pause signal to the remainder of the ophthalmic apparatuses 10. The ophthalmic apparatuses 10 are paused before and during an ophthalmic examination. A specific example during an examination will be described later in Step S107.

In Step S104, following the determination of NO in Step S101, the main body control portion 36 determines whether or not to output a restart signal to the ophthalmic apparatuses 10 in which the interactive function is paused. When a determination of YES is made, the processing proceeds to Step S105, but when a determination of NO is made, the processing proceeds to Step S109. The main body control portion 36 automatically determines whether or not to output a restart signal to the ophthalmic apparatuses 10 whose interactive functions are disabled, upon completion of the ophthalmic examination of the single ophthalmic apparatus 10 selected in Step S102. The restart signal is a signal that restarts the paused interactive function in the ophthalmic apparatus 10 or, in other words, a signal that switches the interactive function from off in a paused state to on again.

A determination of NO in Step S104 means that enable control of the interactive function in the ophthalmic apparatus 10 is continued from Step S52. An example would be a situation where enable control of the interactive function is continued from Step S52 in the first ophthalmic apparatus 10A. In this situation, the disabled or paused state of the interactive function is continued in the remainder of the ophthalmic apparatuses 10.

In Step S105, following the determination of YES in Step S104, the main body control portion 36 determines whether or not the interactive function is paused in the plurality of ophthalmic apparatuses 10. When a determination of YES is made, the processing proceeds to Step S106, but when a determination of NO is made, the processing proceeds to Step S107. At this point, as described in Step S101, the main body control portion 36 can determine whether or not the interactive function is paused in the plurality of ophthalmic apparatuses 10. A determination of YES in Step S105 means that, for example, there are two remaining ophthalmic apparatuses 10 and the interactive function is paused in both ophthalmic apparatuses 10. A determination of NO in Step S105 means that, for example, there are two remaining ophthalmic apparatuses 10 and the interactive function is paused in one of the ophthalmic apparatuses 10.

In Step S106, following the determination of YES in Step S105, the main body control portion 36 selects the ophthalmic apparatus 10 for which the interactive function is to be restarted, and the processing proceeds to Step S107. For example, when there are two remaining ophthalmic apparatuses 10 and the interactive function is paused in both ophthalmic apparatuses 10, the main body control portion 36 selects one of the ophthalmic apparatuses 10 as the ophthalmic apparatus 10 for which the interactive function is to be restarted. The selection of either one is as described in Step S102 and, accordingly, a description thereof will be omitted.

In Step S107, following a determination of NO in Step S105 or a selection of the ophthalmic apparatus 10 in Step S106, the main body control portion 36 outputs a disable signal or a pause signal to the ophthalmic apparatus 10 selected in Step S102 and the processing proceeds to Step S108. For example, the main body control portion 36 outputs the disable signal or the pause signal to the first ophthalmic apparatus 10A selected in Step S102. The main body control portion 36 outputs the disable signal by controlling the function switch 326. The disable signal means that the ophthalmic examination in the first ophthalmic apparatus 10A has been completed. The pause signal means that the ophthalmic examination being executed in the first ophthalmic apparatus 10A will be temporarily suspended. Situations in which the interactive function is temporarily suspended when an ophthalmic examination is in progress include: during an automated examination by the first ophthalmic apparatus 10A that does not require operations by the examiner; during analysis of examination results from the first ophthalmic apparatus 10A; and when the examination is interrupted due to the subject's circumstances.

In Step S108, following the output of a disable signal or a pause signal in Step S107, the main body control portion 36 outputs a restart signal to the ophthalmic apparatus 10 selected in Step S106 or the ophthalmic apparatus 10 whose interactive function is determined to be paused in Step S105, and the processing proceeds to Step S109. The main body control portion 36 outputs a restart signal by controlling the restart switch 328. A restart of the interactive function corresponds to enabling the interactive function. Accordingly, the main body control portion 36 outputs the enable signal by controlling the function switch 326.

In Step S109, following an output of the pause signal in Step S103, a determination of NO in Step S104, or an output of a restart signal in Step S108, the main body control portion 36 determines the following. In Step S109, for all of the three ophthalmic apparatuses 10 connected to the remote operation apparatus 30, the main body control portion 36 determines whether or not the interactive function is disabled. When a determination of YES is made, the main body control portion 36 proceeds to end, but when a determination of NO is made, the main body control portion 36 proceeds to return. At this point, as described in Step S101, the main body control portion 36 can determine whether or not the interactive function is disabled in all of the three ophthalmic apparatuses 10.

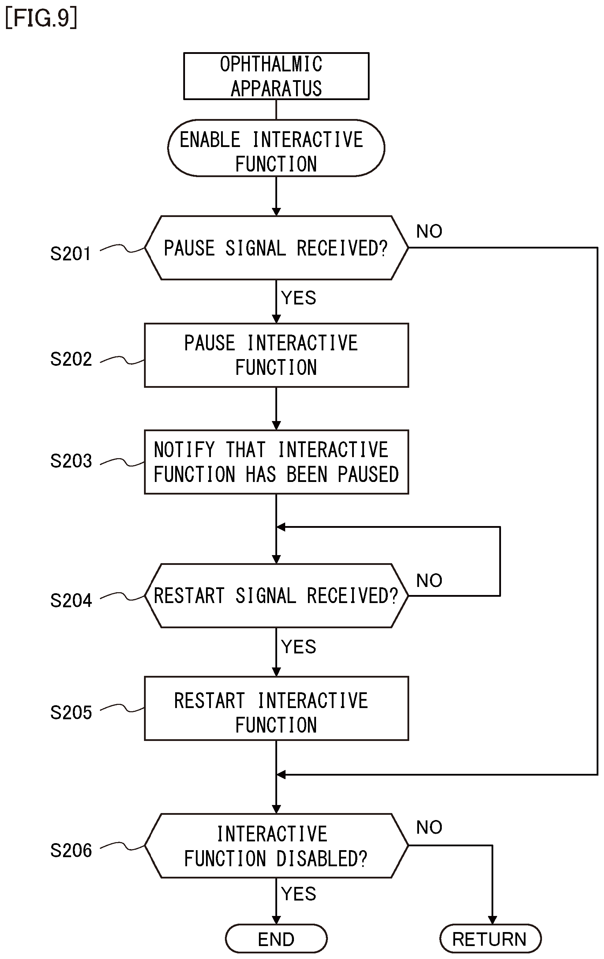

Next, when the interactive function is enabled, FIG. 9 illustrates an example flow of execution performed by the ophthalmic apparatus 10, as described below. The flow chart in FIG. 9 represents processing executed in each ophthalmic apparatus 10 separately from FIGS. 3, 5, and 8 when enable control of the interactive function in Step S52 is executed in each ophthalmic apparatus 10. Steps S201 through S206 constitute processing related to the interactive function which is performed by the ophthalmic apparatus 10 (apparatus control portion 20 and remote operation reception portion 22).

In each ophthalmic apparatus 10, the apparatus control portion 20 executes enable control of the interactive function in Step S52 in response to the main body control portion 36 outputting an enable signal. Accordingly, the flow chart in FIG. 9 starts processing at Step S201. A timing at which the flow chart proceeds to Step S201 is the same as the timing at which the flow chart in FIG. 8 proceeds to Step S101. The execution of enable control of the interactive function in Step S52 by the apparatus control portion 20 in one of the three ophthalmic apparatuses 10 does not guarantee that the flow chart in FIG. 9 proceeds to Step S201 in all three ophthalmic apparatuses 10. In other words, the flow chart in FIG. 9 proceeds to Step S201 in only the ophthalmic apparatus 10 in which enable control of the interactive function in Step S52 was executed by the apparatus control portion 20.

In Step S201, following enable control processing of the interactive function in Step S52 or return, the remote operation reception portion 22 determines whether or not a pause signal has been received. When a determination of YES is made, the processing proceeds to Step S202, but when a determination of NO is made, the processing proceeds to Step S206. When a pause signal is output from the main body control portion 36, the remote operation reception portion 22 receives the pause signal. The remote operation reception portion 22 determines that a pause signal has been received. When the determination by the remote operation reception portion 22 that a pause signal has not been received continues, enable control of the interactive function is to continue from Step S52.

In Step S202, following the reception of the pause signal in Step S201, the remote operation reception portion 22 and the apparatus control portion 20 execute pause processing of the interactive function and the processing proceeds to Step S203. In the pause processing of the interactive function, the remote operation reception portion 22 outputs, to the apparatus control portion 20, the pause signal received in Step S201. Next, by receiving the pause signal, the apparatus control portion 20 performs disable control (audio control) of switching the subject microphone 17 from on to off. Accordingly, the interactive function is paused (temporarily suspended).

In Step S203, following pausing the interactive function in Step S202, the apparatus control portion 20 notifies the subject via the subject speakers 16 that the interactive function has been paused and the processing proceeds to Step S204. In the present processing, when the processing proceeds to Step S203, the subject speakers 16 automatically output automated audio information in response to automatically receiving the automated audio information from the apparatus control portion 20. Alternatively, when the monitor portion 15 receives function notification display information notifying pausing of the interactive function from the apparatus control portion 20, the monitor portion 15 may automatically display the information. An example of the notification in Step S203 is: "The conversation function is currently suspended. Please wait a moment." The "conversation function" is the interactive function.

In Step S204, following the pause notification in Step S203 or the non-reception of a restart signal in Step S204, the remote operation reception portion 22 determines whether or not a restart signal has been received. When a determination of YES is made, the processing proceeds to Step S205, but when a determination of NO is made, the remote operation reception portion 22 repeats Step S204. When a restart signal is output from the main body control portion 36, the remote operation reception portion 22 receives the restart signal. The remote operation reception portion 22 determines that a restart signal has been received. When the determination by the remote operation reception portion 22 that a restart signal has not been received continues, pause of the interactive function is to continue from Step S202.

In Step S205, following the reception of the restart signal in Step S204, the remote operation reception portion 22 and the apparatus control portion 20 execute restart processing (enable control) of the interactive function and the processing proceeds to Step S206. In the restart processing of the interactive function, the remote operation reception portion 22 outputs, to the apparatus control portion 20, the restart signal received in Step S204. Next, by receiving the restart signal, the apparatus control portion 20 performs enable control (audio control) of switching the subject microphone 17 from off to on. Accordingly, the interactive function is enabled.

In Step S206, following non-reception of a pause signal in Step S201 or the restart of the interactive function in Step S205, the apparatus control portion 20 determines whether or not the interactive function is disabled in the ophthalmic apparatus 10. When a determination of YES is made, the processing proceeds to end, but when a determination of NO is made, the processing proceeds to return. The apparatus control portion 20 enables and disables the interactive function in the ophthalmic apparatus 10 and, consequently, stores whether the interactive function of the ophthalmic apparatus 10 is enabled or disabled in a memory (not illustrated). Thus, the apparatus control portion 20 can make the determination at this point.

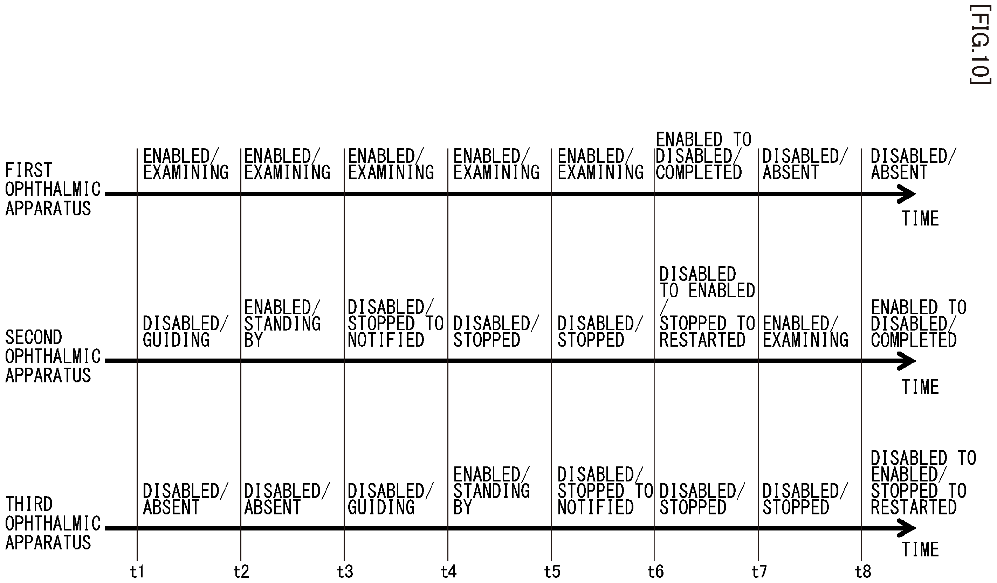

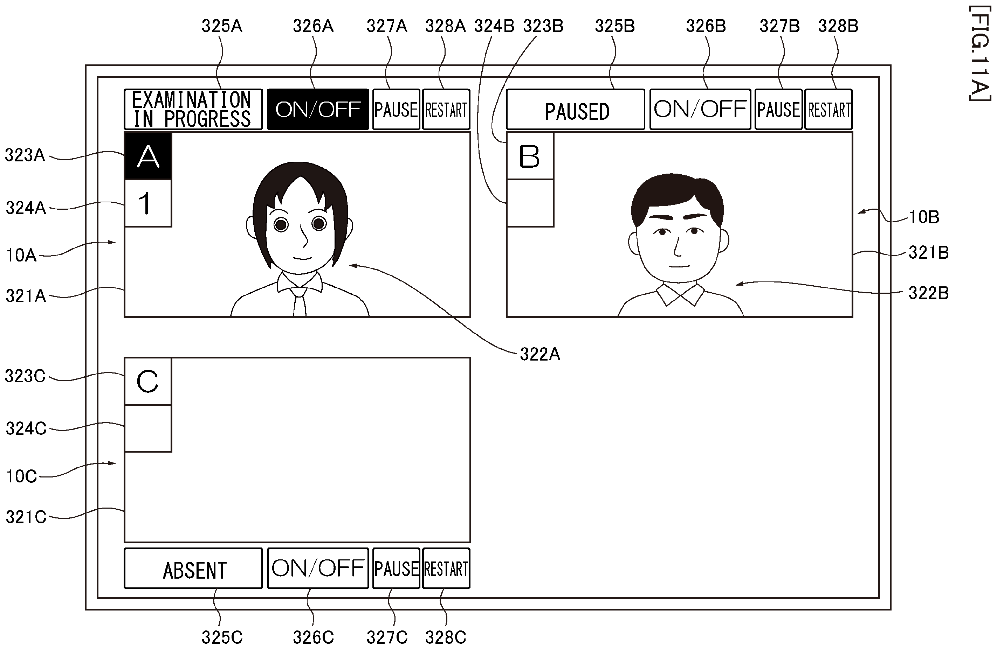

A flow of the flow charts in FIGS. 8 and 9 will be described using display examples of the examiner monitor portion 32 in FIGS. 11A to 11D, based on the exemplary time chart in FIG. 10. In FIG. 10, the horizontal axis represents time and is divided into ophthalmic apparatuses 10 while the vertical axis represents time points (t). A text shown to the right of the vertical line at each time point t in FIG. 10 briefly indicates the state of each ophthalmic apparatus 10 at that time point t. Hereinafter, the texts in FIG. 10 will be described. "Enabled", "Disabled", "Stopped (Paused, Temporarily Suspended)", and "Restarted" indicate states of the interactive function. In addition, "Examining" indicates that an ophthalmic examination of the subject is actually being executed, "Completed" indicates that the ophthalmic examination by the examiner has been completed, and "Absent" indicates that the subject is not present. Furthermore, in FIG. 10, "Guiding" indicates that examination guidance processing is being executed, "Standing By" indicates that the subject is standing by and ready for examination, and "Notified" indicates that the subject is notified that the interactive function is paused. For example, the text "Enabled, Examining" for the ophthalmic apparatus 10 indicates that the interactive function is enabled and that the examination is performed. The arrows indicate a state transition from the left side to the right side of the arrows.

At time point t1, the display on the examiner monitor portion 32 is, for example, in the state shown in FIG. 11A. At time point t1, the first ophthalmic apparatus 10A has the interactive function enabled and performs an ophthalmic examination. At the second ophthalmic apparatus 10B, the subject is present and examination guidance processing is being executed. At the third ophthalmic apparatus 10C, the subject is absent. The interactive function is disabled in the second ophthalmic apparatus 10B and the third ophthalmic apparatus 10C, and none of the three ophthalmic apparatuses 10 have their interactive function paused. Time point t1 corresponds to a flow to "NO" in Step S101, "NO" in Step S104, and "NO" in Step S109 in FIG. 8 in the processing of the main body control portion 36. Time point t1 corresponds to a flow from "NO" in Step S201 to "NO" in Step S206 in FIG. 9 in the processing of the first ophthalmic apparatus 10A. At time point t1, the second ophthalmic apparatus 10B and the third ophthalmic apparatus 10C have the interactive function disabled. For this reason, the processing shown in FIG. 9 is not executed.

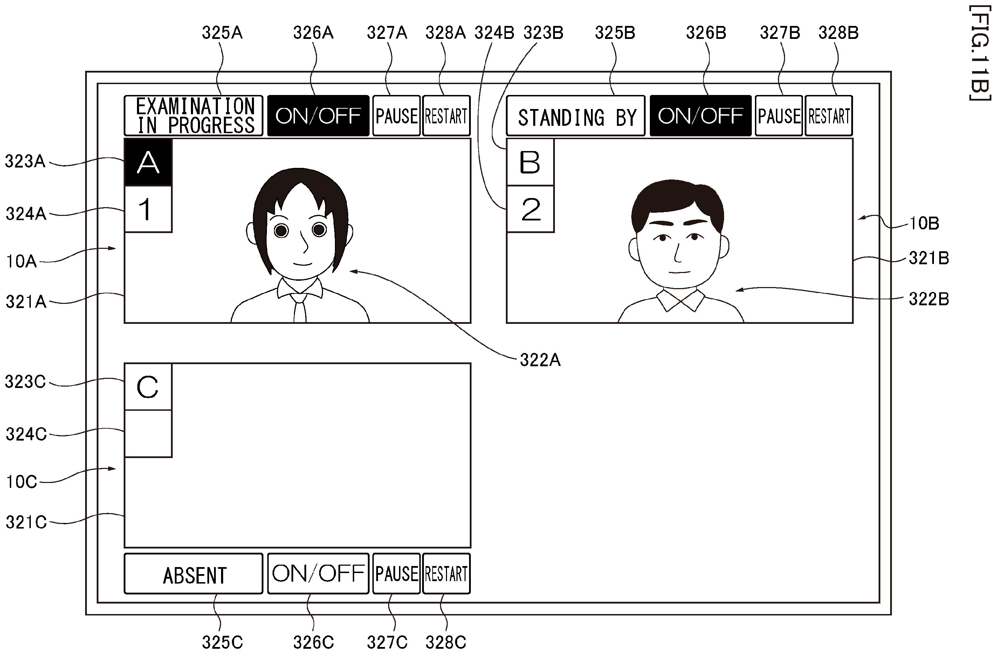

At time point t2, the display on the examiner monitor portion 32 is, for example, in the state shown in FIG. 11B. At time point t2, the states of the first ophthalmic apparatus 10A and the third ophthalmic apparatus 10C are the same as at time point t1. On the other hand, the second ophthalmic apparatus 10B has the interactive function enabled. However, the interactive function of the first ophthalmic apparatus 10A is enabled before the interactive function of the second ophthalmic apparatus 10B. Time point t2 corresponds to a flow from "YES" in Step S101 to Step S102 in FIG. 8 in the processing of the main body control portion 36. Time point t2 corresponds to a flow from "NO" in Step S201 to "NO" in Step S206 in FIG. 9 in the processing of the first ophthalmic apparatus 10A and the second ophthalmic apparatus 10B. At time point t2, the processing shown in FIG. 9 is not executed in the third ophthalmic apparatus 10C in a similar manner to time point t1.

At time point t3, the state of the first ophthalmic apparatus 10A is the same as at time point t1. At the second ophthalmic apparatus 10B, the subject is present but the main body control portion 36 outputs a pause signal. The second ophthalmic apparatus 10B determines that a pause signal has been received. Furthermore, the second ophthalmic apparatus 10B notifies the subject via the subject speakers 16B that the interactive function has been paused. At the third ophthalmic apparatus 10C, the subject is present and examination guidance processing is being executed.

Time point t3 corresponds to a flow from Step S102 to Step S103 in FIG. 8 in the processing of the main body control portion 36. Time point t3 corresponds to a flow from "NO" in Step S201 to "NO" in Step S206 in FIG. 9 in the processing of the first ophthalmic apparatus 10A. Time point t3 corresponds to a flow of "YES" in Step S201, Step S202, Step S203, and "NO" in Step S204 in FIG. 9 in the processing of the second ophthalmic apparatus 10B. In the processing of the second ophthalmic apparatus 10B, the flow of "NO" in Step S204 is repeated until the second ophthalmic apparatus 10B determines that a restart signal has been received. In the processing of the second ophthalmic apparatus 10B, the flow of "NO" in Step S204 is repeated until time point t6 arrives. Accordingly, the description of the processing of the second ophthalmic apparatus 10B will be omitted until time point t6 arrives. At time point t3, the processing shown in FIG. 9 is not executed in the third ophthalmic apparatus 10C in a similar manner to time point t1.

From time point t3 to time point t4, the main body control portion 36 does not output a restart signal to the paused second ophthalmic apparatus 10B. The period from time point t3 to time point t4 corresponds to a flow from Step S103 to "NO" in Step S109 in FIG. 8 in the processing of the main body control portion 36. The period from time point t3 to time point t4 is the same as time point t3 in FIG. 9 in the processing of the first ophthalmic apparatus 10A and the third ophthalmic apparatus 10C.

At time point t4, the state of the first ophthalmic apparatus 10A is the same as at time point t1. The first ophthalmic apparatus 10A and the third ophthalmic apparatus 10C have the interactive function enabled. However, the first ophthalmic apparatus 10A has the interactive function enabled earlier than the third ophthalmic apparatus 10C. Time point t4 corresponds to a flow from "YES" in Step S101 to Step S102 in FIG. 8 in the processing of the main body control portion 36. Time point t4 corresponds to a flow from "NO" in Step S201 to "NO" in Step S206 in FIG. 9 in the processing of the first ophthalmic apparatus 10A and the third ophthalmic apparatus 10C.

At time point t5, the state of the first ophthalmic apparatus 10A is the same as at time point t1. At the third ophthalmic apparatus 10C, the subject is present but the main body control portion 36 outputs a pause signal. The third ophthalmic apparatus 10C determines that a pause signal has been received. Furthermore, the third ophthalmic apparatus 10C notifies the subject via the subject speakers 16C that the interactive function has been paused.

Time point t5 corresponds to a flow from Step S102 to Step S103 in FIG. 8 in the processing of the main body control portion 36. Time point t5 corresponds to a flow from "NO" in Step S201 to "NO" in Step S206 in FIG. 9 in the processing of the first ophthalmic apparatus 10A. Time point t5 corresponds to a flow of "YES" in Step S201, Step S202, Step S203, and "NO" in Step S204 in FIG. 9 in the processing of the third ophthalmic apparatus 10C. In the processing of the third ophthalmic apparatus 10C, the flow of "NO" in Step S204 is repeated until the third ophthalmic apparatus 10C determines that a restart signal has been received. In the processing of the third ophthalmic apparatus 10C, the flow of "NO" in Step S204 is repeated until time point t8 arrives. Accordingly, the description of the processing of the third ophthalmic apparatus 10C will be omitted until time point t8 arrives.

From time point t5 to time point t6, the main body control portion 36 does not output a restart signal to the second ophthalmic apparatus 10B and the third ophthalmic apparatus 10C whose interactive function is paused. The period from time point t5 to time point t6 corresponds to a flow from Step S103 to "NO" in Step S109 in FIG. 8 in the processing of the main body control portion 36. The period from time point t5 to time point t6 is the same as time point t5 in FIG. 9 in the processing of the first ophthalmic apparatus 10A.

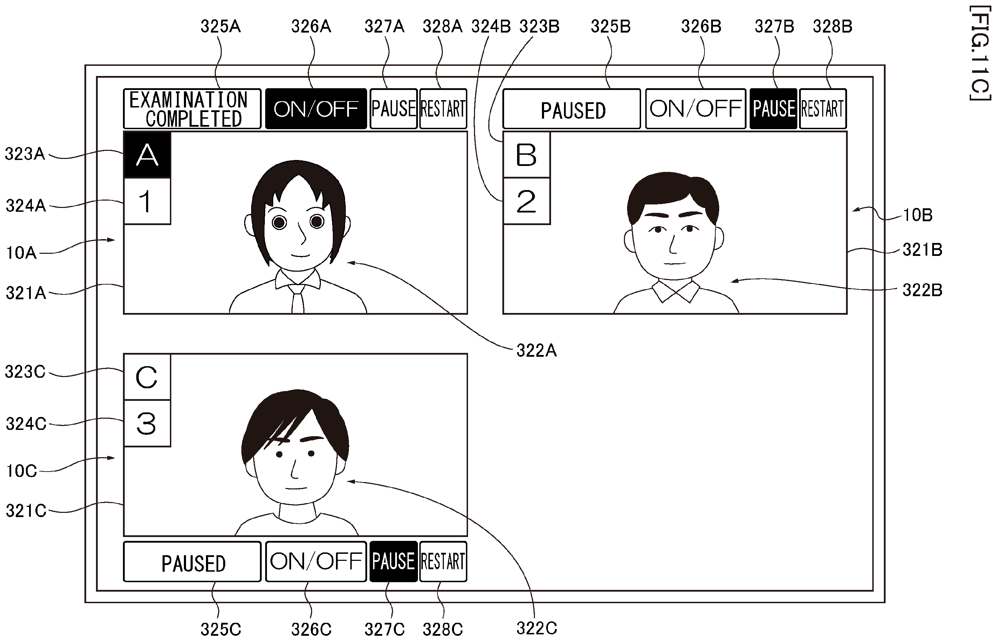

At time point t6, the display on the examiner monitor portion 32 is, for example, in the state shown in FIG. 11C. At time point t6, at the first ophthalmic apparatus 10A, the ophthalmic examination is completed but the interactive function is enabled. The interactive function is paused in the second ophthalmic apparatus 10B and the third ophthalmic apparatus 10C. For this reason, the main body control portion 36 outputs a restart signal to the second ophthalmic apparatus 10B and the third ophthalmic apparatus 10C whose interactive function is paused.

The main body control portion 36 determines that the interactive function is paused in the plurality of ophthalmic apparatuses 10. The second ophthalmic apparatus 10B had the interactive function enabled earlier than the third ophthalmic apparatus 10C. For this reason, the main body control portion 36 selects the second ophthalmic apparatus 10B to restart the interactive function. Next, the first ophthalmic apparatus 10A has the interactive function enabled. For this reason, the main body control portion 36 outputs a disable signal to the first ophthalmic apparatus 10A. Next, the main body control portion 36 outputs a restart signal to the second ophthalmic apparatus 10B. The second ophthalmic apparatus 10B determines that a restart signal has been received. The second ophthalmic apparatus 10B executes processing to restart the interactive function.

Time point t6 corresponds to a flow to "NO" in Step S101, "YES" in Step S104, "YES" in Step S105, Step S106, Step S107, Step S108, and "NO" in Step S109 in FIG. 8 in the processing of the main body control portion 36. Time point t6 corresponds to a flow to "NO" in Step S201, "YES" in Step S206, and end in FIG. 9 in the processing of the first ophthalmic apparatus 10A. Time point t6 corresponds to a flow to "YES" in Step S204, Step S205, and "NO" in Step S206 in FIG. 9 in the processing of the second ophthalmic apparatus 10B.

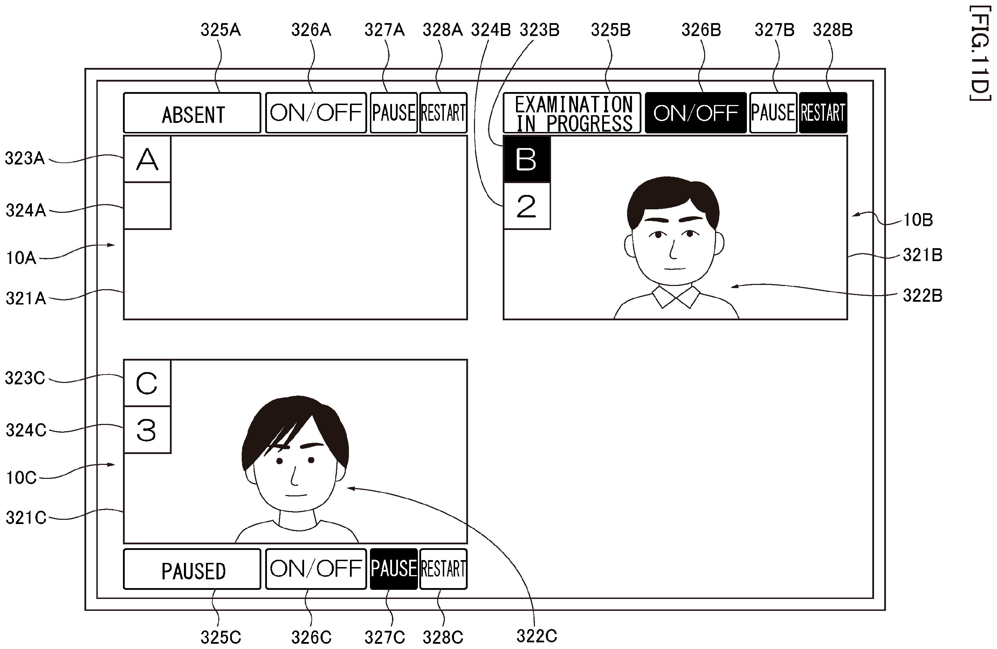

At time point t7, the display on the examiner monitor portion 32 is, for example, in the state shown in FIG. 11D. At time point t7, the subject is absent at the first ophthalmic apparatus 10A. The second ophthalmic apparatus 10B resumes the interactive function, thereby enabling the interactive function and performing an ophthalmic examination. Time point t7 corresponds to a flow to "NO" in Step S101, "NO" in Step S104, and "NO" in Step S109 in FIG. 8 in the processing of the main body control portion 36. At time point t7, the interactive function is disabled in the first ophthalmic apparatus 10A and the processing shown in FIG. 9 is not executed. In addition, the state of the first ophthalmic apparatus 10A remains unchanged after time point t7 and a description will be omitted. Time point t7 corresponds to a flow from "NO" in Step S201 to "NO" in Step S206 in FIG. 9 in the processing of the second ophthalmic apparatus 10B.

At time point t8, the second ophthalmic apparatus 10B completes the ophthalmic examination while maintaining the interactive function enabled. The interactive function is paused in the third ophthalmic apparatus 10C. For this reason, the main body control portion 36 outputs a restart signal to the third ophthalmic apparatus 10C whose interactive function is paused. The main body control portion 36 determines that the interactive function is not paused in the plurality of ophthalmic apparatuses 10. For this reason, the main body control portion 36 selects the third ophthalmic apparatus 10C to restart the interactive function. Next, the second ophthalmic apparatus 10B has the interactive function enabled. For this reason, the main body control portion 36 outputs a disable signal to the second ophthalmic apparatus 10B. Next, the main body control portion 36 outputs a restart signal to the third ophthalmic apparatus 10C. The third ophthalmic apparatus 10C determines that a restart signal has been received. The third ophthalmic apparatus 10C executes processing to restart the interactive function.

Time point t8 corresponds to a flow to "NO" in Step S101, "YES" in Step S104, "NO" in Step S105, Step S107, Step S108, and "NO" in Step S109 in FIG. 8 in the processing of the main body control portion 36. Time point t8 corresponds to a flow to "NO" in Step S201, "YES" in Step S206, and end in FIG. 9 in the processing of the second ophthalmic apparatus 10B. Time point t8 corresponds to a flow to "YES" in Step S204, Step S205, and "NO" in Step S206 in FIG. 9 in the processing of the third ophthalmic apparatus 10C.

After time point t8, the subject at the second ophthalmic apparatus 10B becomes absent. For this reason, the first ophthalmic apparatus 10A and the second ophthalmic apparatus 10B have the interactive function disabled. The third ophthalmic apparatus 10C completes the ophthalmic examination and disables the interactive function. After time point t8 corresponds to a flow to "NO" in Step S201, "YES" in Step S206, and end in FIG. 9 in the processing of the third ophthalmic apparatus 10C. After time point t8 corresponds to a flow from "NO" in Step S101 to "NO" in Step S104, "YES" in Step S109, and end in FIG. 8 in the processing of the main body control portion 36. Accordingly, the processing of the flow charts in FIGS. 8 and 9 is completed.

Next, conventional technical problems that occur when an examiner remotely operates an ophthalmic apparatus using a host apparatus via the Internet or other systems will be described.

Conventionally, in remote operations by the examiner, there are moments where not only the examiner desires to issue instructions to the subject, but also the subject may want to communicate with the examiner. The examiner can remotely operate a plurality of ophthalmic apparatuses using one host apparatus. In other words, one examiner can remotely execute an ophthalmic examination on each of a plurality of subjects. As a result, a problem arises in that the one examiner may need to communicate with the plurality of subjects at the same time. Communicating with a plurality of subjects at the same time makes it difficult for the examiner to communicate appropriately.

Next, the operation of the ophthalmic system 1 according to the first embodiment will be described below.

In the ophthalmic system 1 according to the present disclosure, each ophthalmic apparatus 10 includes the subject microphone 17 and the subject speakers 16. The remote operation apparatus 30 includes the examiner microphone 34 and the examiner speaker 33. The ophthalmic system 1 has an interactive function that enables a subject at each ophthalmic apparatus 10 to communicate with an examiner at the remote operation apparatus 30. When the interactive function is enabled in two or more ophthalmic apparatuses (10A and 10B, 10A and 10C, or 10B and 10C) among three ophthalmic apparatuses 10 (Step S101 in FIG. 8), the remote operation apparatus 30 maintains the enabled state of the interactive function in one ophthalmic apparatus (10A or 10B) (for example, Step S102 in FIG. 8). Furthermore, the remote operation apparatus 30 outputs a pause signal to the remainder of the ophthalmic apparatuses (10B or 10C) (Step S103 in FIG. 8). In other words, when the interactive function is enabled in two or more ophthalmic apparatuses 10, the enabled state of the interactive function is maintained only in any one ophthalmic apparatus 10 (for example, Steps S102 and S103 in FIG. 8). Accordingly, even when one examiner may need to communicate with a plurality of subjects at the same time, the examiner communicates only with the subject being examined by any one of the ophthalmic apparatuses 10. As a result, when the examiner remotely operates three ophthalmic apparatuses 10 using one remote operation apparatus 30, the examiner can individually communicate with three (a plurality of) subjects.

When the interactive function is enabled in two or more ophthalmic apparatuses 10, the remote operation apparatus 30 of the ophthalmic system 1 according to the present disclosure maintains the enabled state of the interactive function in one ophthalmic apparatus 10 for which the interactive function was first enabled (for example, Step S102 in FIG. 8). Thus, the examiner need not select one ophthalmic apparatus 10 capable of communication and the burden of selection on the examiner can be reduced. Furthermore, standby times for ophthalmic examinations can be reduced and processing speeds of ophthalmic examinations can be increased.

The remote operation apparatus 30 of the ophthalmic system 1 according to the present disclosure outputs, to the remainder of the ophthalmic apparatuses 10, a pause signal that pauses the interactive function (Step S103 in FIG. 8). Thus, the interactive function of the remainder of the ophthalmic apparatuses 10 can be reliably paused.

In the ophthalmic system 1 according to the present disclosure, when the remainder of the ophthalmic apparatuses 10 receives the pause signal from the remote operation apparatus 30 (Steps S201 and S202 in FIG. 9), the remainder of the ophthalmic apparatuses 10 notifies the subject at the remainder of the ophthalmic apparatuses 10 that the interactive function has been paused (Step S203 in FIG. 9). Thus, each subject at the remainder of the ophthalmic apparatuses 10 can be reliably notified that the subject cannot communicate with the examiner. As a result, the subject can wait for the ophthalmic examination to start without anxiety.

When the remote operation apparatus 30 of the ophthalmic system 1 according to the present disclosure issues an instruction to disable the interactive function (outputs a disable signal) to one of the ophthalmic apparatuses 10 (Step S107 in FIG. 8), the remote operation apparatus 30 issues an instruction to restart the paused interactive function (outputs a restart signal) to one of the remainder of the ophthalmic apparatuses 10 (Step S108 in FIG. 8). In other words, the interactive function can be restarted for one of the remainder of the ophthalmic apparatuses 10 in place of the one ophthalmic apparatus 10. Thus, the interactive function can be reliably enabled for only one of the remainder of the ophthalmic apparatuses 10.

The remote operation apparatus 30 of the ophthalmic system 1 according to the present disclosure includes the pause switch 327 that outputs a pause signal and the restart switch 328 that outputs a restart signal to each ophthalmic apparatus 10. In other words, the remote operation apparatus 30 can selectively output one of the signals to the ophthalmic apparatuses 10. Thus, the remote operation apparatus 30 can automatically output signals by operating the pause switch 327 and the restart switch 328.

While the ophthalmic system 1 according to the present disclosure has been described above based on the first embodiment, a specific configuration is not limited thereto and design changes or additions are allowed insofar as they do not deviate from the gist of the invention as set forth in each of the claims.

While an example of the main body control portion 36 selecting an ophthalmic apparatus 10 based on the order of the ophthalmic apparatuses 10 in which the subjects started waiting has been described in Step S102 of the first embodiment described above, the selection by the main body control portion 36 is not limited thereto. For example, the main body control portion 36 may select one ophthalmic apparatus 10 that was the first to start communication using the interactive function from among the plurality of ophthalmic apparatuses 10. Specifically, when the interactive function of three ophthalmic apparatuses 10 is enabled, the main body control portion 36 receives subject audio information from among a plurality of ophthalmic apparatuses 10. The main body control portion 36 automatically selects one ophthalmic apparatus 10 for which subject audio information was first input to the main body control portion 36 from the plurality of ophthalmic apparatuses 10. In other words, the main body control portion 36 selects one ophthalmic apparatus 10 based on the subject audio information received from the plurality of ophthalmic apparatuses 10. Thus, given that the communication was the first to be started, the enabled state of the interactive function can be preferentially maintained without interrupting the communication. Furthermore, the examiner need not select one ophthalmic apparatus 10 capable of communication and the burden of selection on the examiner can be reduced.

The examiner may make a selection instead of the selection by the main body control portion 36. The selection by the examiner involves giving priorities to the plurality of ophthalmic apparatuses 10 and selecting one ophthalmic apparatus 10. For example, priorities are given to: subjects with high urgency such as emergency patients; subjects for whom an ophthalmic examination is more necessary than for others based on their condition; or subjects such as children or the elderly who are unlikely to be able to tolerate the waiting time before an ophthalmic examination starts. For the remainder of the ophthalmic apparatuses 10 not selected by the examiner, the interactive function is switched from enabled to paused by selecting the "pause switch 327" shown in FIGS. 11A to 11D. Accordingly, the enabled state of the interactive function in one ophthalmic apparatus 10 is maintained. As a result, for example, when there is a subject with high urgency such as emergency patient, the enabled state of the interactive function can be preferentially maintained by the examiner's selection. When the examiner makes a selection, switching of the interactive function among enabled, disabled, paused, and restarted is executed by the examiner selecting the function switch 326, the pause switch 327, and the restart switch 328.

An example has been described in which the main body control portion 36 outputs a disable signal to the first ophthalmic apparatus 10A at time point t6 in the first embodiment. In addition, an example corresponding to a flow to "NO" in Step S201, "YES" in Step S206, and end in FIG. 9 in the processing of the first ophthalmic apparatus 10A at time point t6 has been described. However, such examples are not restrictive. For example, if the ophthalmic examination at the first ophthalmic apparatus 10A has not been completed, the interactive function may be paused. For this reason, at time point t6, the main body control portion 36 outputs a pause signal to the first ophthalmic apparatus 10A. In addition, time point t6 corresponds to a flow to "NO" in Step S201, "NO" in Step S206, and return in FIG. 9 in the processing of the first ophthalmic apparatus 10A. Subsequently, the interactive function can be restarted in the first ophthalmic apparatus 10A. In this manner, the interactive function can be switched from enabled to paused even during an examination. In other words, as described earlier, pauses include situations during automatic examinations where the first ophthalmic apparatus 10A does not require operations by an examiner. Thus, the interactive function can be switched from enabled to paused even during an ophthalmic examination. In other words, an ophthalmic examination can be continued even if the interactive function is switched from enabled to paused.

While an example in which the function switch 326, the pause switch 327, and the restart switch 328 are displayed on the examiner monitor portion 32 of the first embodiment has been described, the examiner monitor portion 32 is not limited thereto. For example, instead of displaying the restart switch 328, the pause switch 327 is selected when displayed in black letters against a white background. Accordingly, the interactive function may be switched from paused to restarted. In short, regardless of the type and number of switches used, any configuration may be adopted as long as the interactive function can be switched among enabled, disabled, paused, and restarted states.

While an example in which the interactive function is enabled in Step S52 following the execution of the examination briefing in Step S51 in the first embodiment has been described above, the interactive function is not limited thereto. For example, the interactive function may be enabled following the determination that the subject is detected in Step S3 or during the examination guidance processing in Step S4. Alternatively, the interactive function may be enabled during the examination execution processing in Step S5 and before the execution of the examination briefing in Step S51. Accordingly, the subject can convey requests to the examiner before Step S52. Examples of requests include "I need to use the restroom, so could you wait a while?" Even if the interactive function is enabled at some point, the processing shown in FIGS. 8 and 9 will be executed.

While an example in which the lens used in the subject camera 18 is not particularly limited has been described in the first embodiment, the lens is preferably a wide-angle lens and more preferably a fisheye lens. Furthermore, instead of a lens, the subject camera 18 is more preferably a 360-degree camera. Using a wide-angle lens in the subject camera 18 or using a 360-degree camera allows for a wider field of view around the ophthalmic apparatus 10 as compared to using other lenses or similar devices. In other words, the subject camera 18 is capable of capturing images not only of the subject in the examination posture but also of the subject present in the X-direction of the ophthalmic apparatus 10 (or, in the case of a 360-degree camera, around the ophthalmic apparatus 10). As a result, the speed at which the presence of the subject is detected and determined in Step S3 increases, thereby further reducing the time before an ophthalmic examination starts.

While an example in which the apparatus control portion 20 or the main body control portion 36 automatically executes processing in Step S4 and other steps in the first embodiment has been described, automatic processing is not limited thereto. The portions to be processed automatically need not be automated and, instead, the portions may be executed as appropriate via remote operations by the examiner or audio of the examiner. The ophthalmic apparatuses 10 whose interactive function is disabled or paused may be configured so as not to be operated either automatically by the main body control portion 36 or manually by the examiner. Accordingly, the ophthalmic apparatuses 10 whose interactive function is disabled or paused can be prevented from being operated by mistake.

While an example in which the communication interface 21 and the main body control portion 36 exchange various types of information with each other via the router 100 and the Internet 101 has been described in the first embodiment, the example is not restrictive. In short, the communication network connecting the communication interface 21 and the main body control portion 36 need only enable various types of information to be exchanged and enable remote operations by the examiner.

While an example in which the ophthalmic system 1 according to the first embodiment includes three ophthalmic apparatuses 10 and one remote operation apparatus 30 has been described above as shown in FIG. 1 and other drawings, the ophthalmic system 1 is not limited thereto. The ophthalmic apparatus 10 need only be provided in plurality and the ophthalmic system 1 may include two or four or more ophthalmic apparatuses 10. The plurality of ophthalmic apparatuses 10 in Steps S101 and S105 in FIG. 8 are not limited to two, and there may be situations where there are three or more ophthalmic apparatuses 10 in Steps S101 and S105 in FIG. 8 if the ophthalmic system 1 includes four or more ophthalmic apparatuses 10. Even in such situations, a single ophthalmic apparatus 10 can be selected in Steps S102 and S106 in FIG. 8.

The following is further disclosed regarding the descriptions of the embodiment above, and the first to eighth modifications presented below.
(1) An ophthalmic system, including:
   a plurality of ophthalmic apparatuses; and
   a host apparatus provided at a location away from each of the ophthalmic apparatuses,
   wherein each of the plurality of ophthalmic apparatuses and the host apparatus are configured to communicate with one another, and an examiner remotely operates each of the plurality of ophthalmic apparatuses using the host apparatus,
   the plurality of ophthalmic apparatuses and the host apparatus each include a microphone and a speaker,
   the ophthalmic system includes an interactive function that enables a subject at each of the ophthalmic apparatuses and an examiner at the host apparatus to communicate with one another, and
   when the interactive function is enabled in two or more ophthalmic apparatuses among the plurality of ophthalmic apparatuses, the host apparatus is configured to maintain the enabled state of the interactive function in one of the ophthalmic apparatuses and issue an instruction to a remainder of the ophthalmic apparatuses to pause the interactive function thereof.
(2) The ophthalmic system according to (1), wherein when the interactive function is enabled in the two or more ophthalmic apparatuses, the host apparatus is configured to maintain the enabled state of the interactive function in the one ophthalmic apparatus in which the interactive function is first enabled, the one ophthalmic apparatus that first starts communication between the examiner and the subject using the interactive function, or the one ophthalmic apparatus that is selected by the examiner through the host apparatus.
(3) The ophthalmic system according to (1) or (2), wherein the host apparatus is configured to output, to the remainder of the ophthalmic apparatuses, a pause signal that pauses the interactive function.
(4) The ophthalmic system according to (3), wherein the remainder of the ophthalmic apparatuses is configured to notify a subject at the remainder of the ophthalmic apparatuses of a pause of the interactive function when the pause signal is received from the host apparatus.
(5) The ophthalmic system according to any one of (1) to (3), wherein the host apparatus is configured to issue an instruction to one of the remainder of the ophthalmic apparatuses to restart the interactive function, when issuing an instruction to disable or pause the interactive function to the one of the ophthalmic apparatuses.
(6) The ophthalmic system according to any one of (1) to (5), wherein a control portion is configured to switch the interactive function from enabled to disabled once the ophthalmic examination is completed.
(7) The ophthalmic system according to any one of (1) to (6), wherein the host apparatus includes an output portion that is configured to output, to each of the ophthalmic apparatuses, a pause signal that pauses the interactive function and a restart signal that restarts the enabled state from the paused state of the interactive function.
(8) The ophthalmic system according to (7), wherein
   a router apparatus is connected between the plurality of ophthalmic apparatuses and the host apparatus, and
   the host apparatus is configured to output the pause signal or the restart signal to each of the plurality of ophthalmic apparatuses via the router apparatus.

### Cross-references to Related Applications

This application claims priority to Japanese Patent Application No. 2024-002163, filed with the Japan Patent Office on January 10, 2024, the entire disclosure of which is incorporated herein by reference.

## Claims

1. An ophthalmic system, comprising:
a plurality of ophthalmic apparatuses; and
a host apparatus provided at a location away from each of the ophthalmic apparatuses,
wherein each of the plurality of ophthalmic apparatuses and the host apparatus are configured to communicate with one another, and an examiner remotely operates each of the plurality of ophthalmic apparatuses using the host apparatus,
the plurality of ophthalmic apparatuses and the host apparatus each comprise a microphone and a speaker,
the ophthalmic system comprises an interactive function that enables a subject at each of the ophthalmic apparatuses and an examiner at the host apparatus to communicate with one another, and
when the interactive function is enabled in two or more ophthalmic apparatuses among the plurality of ophthalmic apparatuses, the host apparatus is configured to maintain the enabled state of the interactive function in one of the ophthalmic apparatuses and issue an instruction to a remainder of the ophthalmic apparatuses to pause the interactive function thereof.

2. The ophthalmic system according to claim 1, wherein when the interactive function is enabled in the two or more ophthalmic apparatuses, the host apparatus is configured to maintain the enabled state of the interactive function in the one ophthalmic apparatus in which the interactive function is first enabled, the one ophthalmic apparatus that first starts communication between the examiner and the subject using the interactive function, or the one ophthalmic apparatus that is selected by the examiner through the host apparatus.

3. The ophthalmic system according to claim 1, wherein the host apparatus is configured to output, to the remainder of the ophthalmic apparatuses, a pause signal that pauses the interactive function.

4. The ophthalmic system according to claim 3, wherein the remainder of the ophthalmic apparatuses is configured to notify a subject at the remainder of the ophthalmic apparatuses of a pause of the interactive function, when the pause signal is received from the host apparatus.

5. The ophthalmic system according to claim 3, wherein the host apparatus is configured to issue an instruction to one of the remainder of the ophthalmic apparatuses to restart the interactive function when issuing an instruction to disable or pause the interactive function to the one of the ophthalmic apparatuses.

6. The ophthalmic system according to any one of claims 1 to 5, wherein the host apparatus comprises an output portion that is configured to output, to each of the ophthalmic apparatuses, a pause signal that pauses the interactive function and a restart signal that restarts the enabled state from the paused state of the interactive function.
